# EUROPÄISCHE PATENTSCHRIFT

(11) **EP 0 501 921 B1**
(45) Veröffentlichungstag und Bekanntmachung des Hinweises auf die Patenterteilung: **18.07.2001**
(21) Anmeldenummer: 92810128.6
(22) Anmeldetag: 21.02.1992
(51) Int. Cl.: C12N 15/74, C12N 15/09

(54) **Verfahren zur genetischen Manipulation von Myxobakterien**
Process of genetic manipulation of Myxobacteria
Procédé de manipulation génétique de la Myxobactérie

(30) Priorität: 01.03.1991 CH 62691
(43) Veröffentlichungstag der Anmeldung: 02.09.1992
(73) Patentinhaber: Syngenta Participations AG, 4058 Basel (CH)
(72) Erfinder: Jaoua, Samir, Sfax (TN); Schupp, Thomas, Dr., CH-4313 Möhlin (CH); Neff, Snezana, CH-4416 Bubendorf (CH)

(56) Entgegenhaltungen:
- EP-A- 0 317 509
- EP-A- 0 372 230
- MGG MOLECULAR AND GENERAL GENETICS Bd. 211, Nr. 1, 1988, BERLIN D Seiten 63 - 71; SHIMKETS L J;ASHER S J: 'USE OF RECOMBINATION TECHNIQUES TO EXAMINE THE STRUCTURE OF THE CSG LOCUS OF MYXOCOCCUS-XANTHUS'
- Reichenbach, H. und Dworkin, M., The Order Myxobacterales in The Prokaryotes, Springer Verlag, Vol. 1 (1981), Editor: Starr, M.P. et al.

## Beschreibung

Die vorliegende Erfindung betrifft ein neuartiges Verfahren zur genetischen Manipulation von Myxobakterien aus der *Sorangium/Polyangium* Gruppe, wodurch erstmals eine gezielte Anwendung rekombinanter DNA Techniken auf diese Organismengruppe ermöglicht wird.

Insbesondere betrifft die vorliegende Erfindung ein Verfahren zur Einschleusung von DNA-Sequenzen homologen oder heterologen Ursprungs oder einer Kombination von DNA-Sequenzen homologen und heterologen Ursprungs in das Chromosom besagter Myxobakterien via homologer Rekombination sowie mit Hilfe dieses Verfahrens hergestellte, genetisch modifizierte Myxobakterien.

Ebenfalls umfasst sind rekombinante DNA Moleküle, Plasmide und Vektoren, die für eine Verwendung in dem erfindungsgemässen Verfahren besonders angepasst sind sowie genetisch modifizierte Myxobakterien aus der *Sorangium/Polyangium* Gruppe enthaltend exogene DNA homologen und/oder heterologen Ursprungs.

Bei den Myxobakterien aus der *Sorangium/Polyangium* Gruppe handelt es sich um hochspezialisierte Organismen, die sich häufig in Erdproben, abgestorbenem Pflanzenmaterial oder in Tier-Dung nachweisen lassen. Charakteristisch für diese Gruppe von Mikroorganismen ist ihre Fähigkeit Cellulose oder Cellulose-haltige Abbauprodukte als einzige C-Quelle zu nutzen. Ein weiteres charakteristisches Merkmal dieser Gruppe ist ihre Fähigkeit zur Herstellung hochaktiver Sekundärmetaboliten.

Mittlerweile sind zahlreiche Stämme aus dieser Gruppe beschrieben, die beispielsweise in der Lage sind pflanzenmikrobizide Verbindungen zu synthetisieren. Von besonderer Bedeutung sind dabei die sog. Soraphene, makrocyclische Verbindungen, die ein günstiges biozides Spektrum gegenüber phytopathogenen Mikroorganismen, insbesondere aber gegenüber phytopathogenen Pilzen aufweisen. Diese Verbindungen besitzten sehr vorteilhafte kurative, systemische und insbesondere präventive Eigenschaften und lassen sich zum Schutz von zahlreichen Kulturpflanzen einsetzen [EP 0 358 606].

Auch von anderen Vertretern aus der Gruppe der Myxobakterien ist bekannt, dass sie in der Lage sind hochaktive Verbindungen mit antibiotischer Potenz zu synthetisieren [Reichenbach *et al* (1988)]. Aufgrund der Bedeutung dieser Verbindungen besteht ein grosses Interesse daran, die genetischen Grundlagen ihrer Synthese zu verstehen um somit die Möglichkeit dafür zu schaffen, diese gebenenfalls gezielt beeinflussen zu können.

Voraussetzung dafür ist die Bereitstellung eines Verfahrens, das eine direkte und vorzugsweise zielgerichtete genetische Manipulation dieser Organismen unter Verwendung rekombinanter DNA Techniken ermöglicht, beispielsweise durch den zielgerichteten Einbau von neuen Genen oder Genfragmenten oder sonstigen DNA-Sequenzen, einschliesslich ganzer Plasmide, in das Genom der Myxobakterien.

Verfahren zum konjugativen Transfer von mobilisierbaren Vektoren aus *E. coli* in Gram-positive Bakterien, insbesondere Corynebakterien, sind in EP 0372 230 beschrieben.

Auch einzelne Vertreter aus der Gruppe der Myxobakterien waren bereits früher Gegenstand von Untersuchungen in dieser Richtung. Das besonderes Interesse galt dabei in erster Linie *Myxococcus xanthus*, einem mittlerweile bereits recht gut erforschten Myxobakterium, für das auch schon verschiedene Gentransfer-Verfahren beschrieben sind. So wurde beispielsweise der Coliphage P1 sehr intensiv genutzt, zunächst für die Einschleusung von Transposon Tn5 in das *Myxococcus xanthus* Chromosom [Kaiser (1984); Kuner und Kaiser (1981)] und später dann für den Rücktransfer von in *Myxococcus xanthus* klonierten Genen auf den ursprünglichen E. *coli* Wirt [O'Conner und Zusman (1983); Shimkets *et al* (1983)].

Ein anderes Beispiel betrifft die genetische Manipulation von *Myxococcus xanthus* via Transduktion (Shimkets und Asher, 1988).

Ein weiteres Verfahren zum Gentranfer beruht auf der Verwendung des Plasmids RP4, welches einen sehr weiten Wirtsbereich aufweist. Breton *et al* (1985) konnten zeigen, dass sich dieses Plasmid *via* Konjugation von *E*. *coli* auf *Myxococcus xanthus* übertragen lässt und dort stabil in das Chromosom integriert wird. Basierend auf diesen Eigenschaften gelang es Breton *et al* (1986) sowie Breton und Guespin-Michel (1987) Fremdgene in das Chromosom von *Myxococcus xanthus* zu integrieren. Wie aus Untersuchungen von Jaoua *et al* (1987; 1989) hervorgeht, beruht die beobachtete Integration mit grosser Wahrscheinlichkeit auf einer sog. 'site specific recombination' . Diese ist beschränkt auf bestimmte, räumlich eng begrenzte Stellen innerhalb des *Myxococcus xanthus* Chromosoms und wird vermittelt über einen oder mehrere sog. 'hot spots' auf dem RP4 Plasmid. Darüberhinaus hat sich im Verlaufe der im Rahmen der vorliegenden Erfindung durchgeführten Untersuchungen gezeigt, dass das hier diskutierte, vorbekannte *Myxococcus-System* auf Bakterien aus der *Sorangium/Polyangium* Gruppe nicht anwendbar ist. Vermutlich fehlen diesen Organismen die für eine 'site specific recombination' notwendigen, spezifschen Strukturelemente auf ihren Chromosomen. Darüberhinaus hat man gefunden, dass bei diesen Organismen auch keine stabile Transposition, beispielsweise bei Verwendung von Transposon Tn5, stattfindet.

Die Aufgabe die es im Rahmen dieser Erfindung zu lösen galt betraf somit in erster Linie die Bereitstellung eines universell anwendbaren Verfahrens zur genetischen Manipulation von Myxobakterien aus der *SorangiumlPolyangium* Gruppe, das frei ist von den zuvor genannten Beschränkungen der bekannten Verfahren und das somit eine ungerichtete oder aber vorzugsweise eine zielgerichtete Einschleusung von genetischem Material in Myxobakterien erlaubt, unabhängig von vorgegebenen Strukturelementen auf dem Myxobakterienchromosom oder von spezifischen Transpositionsereignissen.

Dies kann erfindungsgemäss beispielsweise dadurch erreicht werden, dass man genetische Konstrukte herstellt, insbesondere Plasmid-Vektoren, die aufgrund ihres spezifischen Aufbaus an beliebigen Orten innerhalb des Chromosoms inserieren können und die nicht, wie dies bei den vorbekannten Verfahren der Fall ist, an bestimmte, durch die funktionelle Organisation des Myxobakterium-Chromosoms oder des verwendeten Plasmids ('hot spots') vorgebene Integrationsorte auf dem Genom gebunden oder aber von Transpositionen integrierter Transposons abhängig sind.

Die vorliegende Erfindung betrifft somit in erster Linie ein Verfahren zur genetischen Manipulation von Myxobakterien aus der *Sorangium/Polyangium* Gruppe, das sich dadurch kennzeichnet, dass man genetisches Material homologen oder heterologen Ursprungs oder eine Kombination von genetischem Material homologen sowie heterologen Ursprungs in die Myxobakterienzelle einschleust und *via* homologer Rekombination zufallsmässig oder aber, bei entsprechender Kenntnis der strukturellen und funktionellen Organisation des bakteriellen Genoms, gezielt an einer aufgrund der zwischen eingeschleuster DNA und bakterieneigener DNA vorhandenen Homologie genau definierten Stelle in das Chromosom besagter Myxobakterien integriert, unabhängig von vorgegebenen Strukturelementen auf dem Myxobakterienchromosom oder von spezifischen Transpositionsereignissen.

Das erfindungsgemässe Verfahren zur genetischen Manipulation von Myxobakterien aus der Sorangium/Polyangium Gruppe ist insbesondere dadurch gekennzeichnet, dass man
(a) genetisches Material homologen oder heterologen Ursprungs oder eine Kombination von genetischem Material homologen sowie heterologen Ursprungs, das zu einem korrespondierenden Bereich auf dem Myxobakterienchromosom homolog oder aber zumindest im wesentlichen homolog ist; oder aber
(b) genetisches Material, welches natürlicherweise keine zu einem korrespondierenden Bereich auf dem Myxobakterienchromosom homologen oder aber zumindest im wesentlichen homologen Abschnitte enthält und welches daher artifiziell mit Hilfe an sich bekannter rDNA Techniken mit solchen homologen oder aber im wesentlichen homologen DNA Abschnitten verknüpft ist, wodurch das einzuschlensende genetische material von besagten homologen oder aber im wesentlichen homologen DNA-Abschnitten flankiert wird,
in die Myxobakterienzelle einschleust und dort via homologer Rekombination an einer aufgrund der zwischen eingeschleuster DNA und bakterieneigener DNA vorhandenen Homologien genau definierten Stelle in das Chromosom besagter Myxobakterien integriert.

Die Integration des genetischen Materials in das Bakterienchromosom via homologer Rekombination wird durch einen oder mehrere DNA Abschnitte innerhalb der einzuschleusenden DNA vermittelt, die einem korrespondierenden Bereich auf dem Myxobakterienchromosom homolog oder aber zumindest im wesentlichen homolog sind. Sie ist damit nicht an bestimmte chromosomale Strukturen gebunden sondern kann im Prinzip an jeder beliebigen Stelle innerhalb des bakteriellen Chromosoms erfolgen.

Die im Rahmen des erfindungsgemässen Verfahrens verwendbaren homologen DNA Abschnitte müssen somit nicht notwendigerweise 100%ige Identität mit den entsprechenden Abschnitten auf dem Myxobakterienchromosom aufweisen um das geforderte Rekombinationsereignis herbeiführen zu können. Es ist vielmehr ausreichend, wenn diese DNA Abschnitte den korrespondieren Bereichen auf dem Bakteriengenom im wesentlichen homolog sind, d.h. wenn diese einen Homologiegrad zwischen 80% und 100% und ganz besonders bevorzugt zwischen 90% und 100% aufweisen.

Unter "homologen" DNA Abschnitten sollen im folgenden daher auch solche Abschnitte verstanden werden, die zu den korrespondierenden Bereichen auf dem Myxobakterienchromosom keine 100%ige Identität aufweisen, diesen aber zumindest "im wesentlichen homolog" sind.

Die homologen DNA Abschnitte können dabei entweder aus dem Zielorganismus selbst oder aber aus verwandten Organismen, beispielsweise nach Fragmentierung des jeweiligen Genoms, isoliert werden. Bei Kenntnis der DNA-Sequenz der jeweils für die Integration vorgesehenden DNA Abschnitte auf dem Myxobakterienchromosom, können die entsprechenden DNA Fragmente, die diesen chromosomalen Abschnitten homolog oder aber zumindest im wesentlichen homolog sind, selbstverständlich auch synthetisch hergestellt werden.

Bei den im Rahmen der vorliegenden Erfindung verwendbaren DNA-Abschnitten homologen Ursprungs kann es sich dabei entweder um DNA-Abschnitte bekannter Sequenz oder aber um zufällig, beispielsweise nach Restriktionsverdauung homologer DNA, erhältliche DNA-Fragmente handeln.

Bei Kenntnis der strukturellen und funktionellen Organisation der entsprechenden Teile des bakteriellen Genoms eröffnet das erfindungsgemässe Verfahren damit erstmals die Möglichkeit einer zielgerichteten, vorhersagbaren Modifikation der auf dem Myxobakteriengenom vorliegenden Gene (*in situ*-Modifikation), durch Austausch von natürlichen oder artifiziell modifizierten Genen, Genfragmenten oder sonstigen nützlichen DNA-Sequenzen mit homologen DNA-Abschnitten innerhalb des bakteriellen Genoms. Weiterhin ermöglicht das erfindungsgemässe Verfahren eine gezielte Identifikation der Funktion von Einzelgenen innerhalb des Gesamtgenoms von Myxobakterien aus der Sorangium/Polyangium Gruppe durch gezieltes Ausschalten von Genen ['gene disruption'] oder durch Komplementation von Genen, die zuvor durch Anwendung anderer Verfahren, insbesondere von Mutationsverfahren, inaktiviert worden sind.

Neben der zielgerichteten und damit sehr effizienten *in situ* Modifikation von bakterien-eigenen Genen (gezielte Mutagenese), eröffnet das erfindungsgemässe Verfahren eine Reihe weiterer Anwendungsmöglichkeiten, wie z.B. den Einbau zusätzlicher Genkopien in Regionen mit bekannt hoher Expressionsrate sowie die Elimination und damit Ausschaltung von unerwünschten Genen. Darüberhinaus können nunmehr auch die folgenden weiteren Anwendungsmöglichkeiten ins Auge gefasst werden.
- Einbau von starken oder regulierbaren Promotoren vor bakterieneigenen Genen mit interessanten Funktionen.
- Klonierung von Genen verschiedenen Ursprungs in Myxobakterien aus der *Sorangium/Polyangium* Gruppe.
- Klonierung und Expression von Genen verschiedenen Ursprungs.
- Rücktransfer der eingeschleusten DNA in einen anderen Mikroorgansimus wie z.B. in E. *coli* zur weiteren Bearbeitung.
- Einsatz der heterologen DNA eines eingesetzten Vektors erstens als radioaktive Sonde zum Erkennen der dem Integrationsort benachbarten Myxobakterium-Fragmente und zweitens als Vorlage ['Template'] für eine Amplifikation im Rahmen einer "Polymerase Chain Reaction" [PCR].

Ein weiterer Gegenstand der vorliegenden Erfindung betrifft ein Verfahren zur Herstellung genetisch modifizierter Bakterien aus der *Sorangium*/*Polyangium* Gruppe, das sich dadurch kennzeichnet, dass man
(a₁) genetisches Material homologen oder heterologen Ursprungs oder eine Kombination von genetischem Material homologen sowie heterologen Ursprungs, das zu einem korrespondierenden Bereich auf dem Myxobakterienchromosom homolog oder aber zumindest im wesentlichen homolog ist, oder aber
(a₂) genetisches Material, welches natürlicherweise keine zu einem korrespondierenden Bereich auf dem Myxobakterienchromosom homologen oder aber zumindest im wesentlichen homologen Abschnitte enthält und daher artifiziell mit Hilfe an sich bekannter rDNA Techniken mit solchen homologen oder aber im wesentlichen homologen DNA Abschnitten verknüpft ist, wodurch das einzuschleusende genetische Material von besagten homologen oder aber im wesentlichen homologen DNA-Abschnitten flankiert wird;
   in die Myxobakterienzelle einschleust und dort via homologer Rekombination an einer aufgrund der zwischen eingeschleuster DNA und bakterieneigener DNA vorhandenen Homologien genau definierten Stelle in das Chromosom besagter Myxobakterien integriert und
(b) positive Transformanten mit Hilfe an sich bekannter Selektionsverfahren selektiert und als Reinkultur kultiviert.

Die vorliegende Erfindung erlaubt somit erstmals eine zielgerichtete genetische Manipulation des Genoms von Myxobakterien aus der *Sorangium/Polyangium* Gruppe, wobei die Integration des genetischen Materials abhängig von der jeweiligen Zielrichtung des geplanten Vorgehens wahlweise entweder durch homologe DNA-Abschnitte bekannter Sequenz oder aber durch zufallsmässig ausgewählte, homologe Abschnitte unbekannter Sequenz vermittelt werden kann.

Ebenfalls umfasst von der vorliegenden Erfindung sind rekombinante DNA Moleküle, die aufgrund ihrer spezifischen Konstruktion in der Lage sind genetisches Material, wie z.B. Gene oder Genfragmente oder sonstige nützliche DNA-Sequenzen, die gegebenenfalls für neue und wünschenswerte Eigenschaften kodieren, mit Hilfe der homologen Rekombination zufallsgemäss oder aber, bei Kenntnis der strukturellen und funktionellen Organisation des bakteriellen Genoms, auch zielgerichtet an aufgrund der vorhandenen Homologien zwischen eingeschleuster DNA und bakterieneigener DNA genau definierten Stellen innerhalb des bakteriellen Genoms zu integrieren sowie Verfahren zur Herstellung besagter rekombinanter DNA Moleküle.

Weiterhin umfasst von der vorliegenden Erfindung sind genetisch modifizierte Myxobakterien aus der *Sorangium/Polyangium* Gruppe mit gegebenenfalls neuen und/oder verbesserten Eigenschaften, die durch Einschleusen besagter rekombinanter DNA-Moleküle hergestellt worden sind.

Die vorliegende Erfindung betrifft ausserdem die Nachkommen besagter modifizierter Myxobakterien sowie Mutanten und Varianten davon, die noch besagtes rekombinantes DNA Molekül enthalten.

In der folgenden Beschreibung werden eine Reihe von Ausdrücken verwendet, die in der rekombinanten DNA Technologie, sowie in der Bakteriengenetik gebräuchlich sind.

Um ein klares und einheitliches Verständnis der Beschreibung und der Ansprüche sowie des Umfangs, der den besagten Ausdrücken zukommen soll, zu gewährleisten, werden die folgenden Definitionen aufgestellt:

**Gen(e) oder DNA heterologen Ursprungs:** Eine DNA Sequenz, die für ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA oder als exogene DNA bezeichnet.

**Gen(e) oder DNA homologen Ursprungs:** Eine DNA Sequenz, die für ein spezifisches Produkt oder Produkte kodiert oder eine biologische Funktion erfüllt und die aus der gleichen Spezies stammt, in welche das besagte Gen eingeschleust wird. Auch diese DNA wird als exogene DNA bezeichnet.

**DNA Homologie:** Grad der Übereinstimmung zwischen zwei oder mehreren DNA Sequenzen.

**Synthetische(s) Gen(e) oder DNA:** Eine DNA-Sequenz, die für ein spezifisches Produkt oder Produkte kodiert oder eine biologische Funktion erfüllt und die auf synthetischem Wege hergestellt ist.

**Promotor:** Eine Kontrollsequenz der DNA Expression, die die Transkription jeder beliebigen homologen oder heterologen DNA Gensequenz in einer Wirtszelle gewährleistet, sofern besagte Gensequenz in operabler Weise mit einem solchen Promotor verknüpft ist und dieser in besagter Wirtszelle aktiv ist.

**Terminations-Sequenz:** DNA-Sequenz am Ende einer Transkriptions-Einheit, die das Ende des Transkriptionsvorganges signalisiert.

**Ueberproduzierender Promotor (OPP):** Promotor, der in der Lage ist, in einer Wirtszelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.

**3'/5' nicht-translatierte Region:** Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B. die Ribosomenbindungsstelle (5').

**DNA-Expressions-Vektor:** Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

**DNA-Transfer-Vektor:** Transfer-Vehikel, wie z.B. ein Plasmid oder ein Bakteriophagen-Vektor, der die Einschleusung von genetischem Material in eine geeignete Wirtszelle ermöglicht.

**Homologe Rekombination:** reziproker Austausch von DNA Stücken zwischen homologen DNA-Molekülen.

**Mutanten, Varianten:** Spontan oder aber artifiziell, unter Anwendung bekannter Verfahrensmassnahmen, wie z.B. UV-Behandlung, Behandlung mit mutagenen Agentien etc. entstandener Abkömmling eines Mikroorganismus, der noch die erfindungswesentlichen Merkmale und Eigenschaften des Ausgangsstammes aufweist, welcher dieser aufgrund der Transformation mit exogener DNA erhalten hat.

Im Rahmen der vorliegenden Erfindung ist es jetzt erstmals gelungen, ein Verfahren zur Verfügung zu Stellen, das eine vorzugsweise zielgerichtete genetische Manipulation des Genoms von Myxobakterien aus der *Sorangium/Polyangium*-Gruppe ermöglicht, indem nunmehr genetisches Material zufallsgemäss oder aber, bei entsprechender Kenntnis der strukturellen und funktionellen Organisation des bakteriellen Genoms, gezielt an genau definierten, z.T. vorherbestimmbaren Positionen innerhalb des bakteriellen Genoms integriert und dort auch exprimiert werden kann, unabhängig von vorgegebenen Strukturelementen auf dem Myxobakterienchromosom oder von spezifischen Transpositionsereignissen.

Das erfindungsgemässe Verfahren basiert dabei im wesentlichen auf der Erkenntnis, dass es möglich ist, exogenes genetisches Material mit Hilfe der homologen Rekombination in das Genom der Myxobakterien einzubauen, wobei neben natürlichen auch artifiziell modifzierte und/oder synthetische Gene oder Genfragmente oder sonstige DNA-Sequenzen einschliesslich ganzer Plasmide verwendet werden können, sofern sie DNA-Abschnitte besitzen oder von solchen DNA-Abschnitten flankiert werden, die eine für eine Rekombinantion ausreichende Homologie zu korrespondieren Abschnitten auf dem Myxobakteriengenom aufweisen.

Die im Rahmen des erfindungsgemässen Verfahrens verwendbaren homologen DNA Abschnitte müssen dabei nicht notwendigerweise 100%ige Identität mit den entsprechenden Abschnitten auf dem Myxobakterienchromosom aufweisen um das geforderte Rekombinationsereignis herbeiführen zu können. Es ist vielmehr ausreichend, wenn diese DNA Abschnitte den korrespondieren Bereichen auf dem Bakteriengenom im wesentlichen homolog sind, d.h. wenn diese einen Homologiegrad zwischen 80% und 100% und ganz besonders bevorzugt zwischen 90% und 100% aufweisen.

Die Grösse besagter homologer Bereiche kann variieren, sollte aber wenigstens 100 Bp ausmachen. Bevorzugt im Rahmen dieser Erfindung sind Homologiebereiche, die zwischen 0,3 Kb und 4 Kb, vorzugsweise aber zwischen 1 Kb und 3 Kb umfassen.

Einen wesentlichen Bestandteil der vorliegenden Erfindung bilden rekombinante DNA-Moleküle, die aufgrund ihrer spezifischen Konstruktion den gezielten Einbau von Genen oder Genfragmenten oder sonstigen interessierenden DNA-Sequenzen, einschliesslich ganzer Plasmide, in das Genom einer Zielzelle mit Hilfe der homologen Rekombination in der zuvor genannten Weise ermöglichen.

Insbesondere betrifft die vorliegende Erfindung rekombinante DNA Moleküle, die eine zielgerichtete Integration von genetischem Material wie z.B. Genen, Genfragmenten oder sonstigen DNA-Fragmenten an einer definierten Stelle innerhalb des Genoms von Myxobakterien aus der *Sorangium/Polyangium* Gruppe ermöglichen und die sich dadurch kennzeichnen, dass sie die zu integrierende DNA enthalten, und dass besagte DNA in einem Masse Homologien zu entsprechenden DNA Regionen innerhalb des myxobakteriellen Genoms aufweist oder aber von solchen homologen DNA-Sequenzen flankiert wird, dass bei der Transformation der die homologen DNA Regionen enthaltenden Myxobakterien-Zelle eine ungerichtete oder aber vorzugsweise eine zielgerichtete Integration besagter zu integrierender DNA an einer aufgrund der zwischen eingeschleuster DNA und bakterieneigener DNA vorhandenen Homologie genau definierten Stelle innerhalb des Myxobakteriengenoms *via* homologer Rekombination stattfindet.

Besagte rekombinante DNA Moleküle lassen sich sehr einfach herstellen, indem man die zu integrierende DNA, welche die oben genannten Eigenschaften aufweist,
(a) aus einer geeigneten Quelle isoliert; oder
(b) sofern besagte zu integrierende DNA natürlicherweise keine zu einem korrespondierenden Bereich auf dem Myxobakterienchromosom homologen oder aber zumindest im wesentlichen homologen Abschnitte enthält, diese artifiziell mit Hilfe an sich bekannter rDNA Techniken mit entsprechenden homologen oder aber im wesentlichen homologen DNA-Abschnitten verknüpft, wodurch das einzuschlensende genetische Material von besagten homologen oder aber im wesentlichen homologen DNA-Abschnitten flankiert wird.

Sofern es sich bei der zu integrierenden DNA um eine exprimierbare DNA-Sequenz handelt, ist es vorteilhaft, wenn diese in operabler Weise mit in der bakteriellen Zelle funktionsfähigen Expressionssignalen verknüpft ist sowie gegebenfalls von DNA-Abschnitten, die Homologien zu einer bestimmten Region innerhalb des bakteriellen Genoms aufweisen, flankiert wird. Diese flankierenden, homologen DNA-Abschnitte liegen vorzugsweise miteinander zu einer Einheit verschmolzen als Bestandteil ringförmig geschlossener DNA-Moleküle vor.

Letzteres kann entfallen, sofern besagte exprimierbare DNA-Sequenz bereits selbst eine genügend grosse Homologie zu entsprechenden DNA Regionen innerhalb des bakteriellen Genoms aufweist, sodass ein direkter Austausch dieser DNA-Sequenz gegen besagte homologe genomische DNA vermittels der homologen Rekombination erfolgen kann.

Neben doppelsträngiger DNA kann in dem erfindungsgemässen Verfahren auch einzelsträngige DNA sowie teilweise einzelsträngige DNA eingesetzt werden.

Für die Verwendung in dem erfindungsgemässen Verfahren kommen sowohl homologe als auch heterologe Gen(e) oder DNA-Sequenzen sowie synthetische Gen(e) oder DNA-Sequenzen gemäss der im Rahmen der vorliegenden Erfindung gemachten Definition in Betracht.

Die zu integrierenden DNA-Sequenzen können dabei ausschliesslich aus genomischer, aus cDNA bzw. synthetischer DNA konstruiert sein. Eine andere Möglichkeit besteht in der Konstruktion von hybriden DNA-Sequenzen, bestehend sowohl aus cDNA als auch aus genomischer DNA und/oder synthetischer DNA.

In diesem Fall kann die cDNA aus demselben Gen oder DNA-Abschnitt stammen wie die genomische DNA oder aber, sowohl die cDNA, wie auch die genomische DNA können aus verschiedenen Genen oder DNA-Abschnitten stammen. In jedem Fall können aber sowohl die genomischen DNA und/oder die cDNA, jede für sich, aus dem gleichen oder aus verschiedenen Genen oder DNA-Abschnitten hergestellt sein.

Wenn die DNA-Sequenz Anteile von mehr als einem Gen oder DNA-Abschnitt beinhaltet, können diese entweder von ein und demselben Organismus, von mehreren Organismen, die verschiedenen Stämmen, oder Varietäten derselben Art oder verschiedenen Spezies derselben Gattung angehören, oder aber von Organismen, die mehr als einer Gattung derselben oder einer anderen taxonomischen Einheit angehören, abstammen.

Um die Expression eines Strukturgens in der bakteriellen Zelle zu gewährleisten, können die kodierenden Gensequenzen gegebenenfalls zunächst in operabler Weise mit in der Myxobakterienzelle funktionsfähigen Expressions-Sequenzen verknüpft werden.

Die exprimierbaren hybriden Genkonstruktionen der vorliegenden Erfindung enthalten somit in der Regel neben dem (den) Strukturgen(en) auch Expressions-Signale, die sowohl Promoter- und Terminator-Sequenzen als auch vorzugsweise weitere regulatorische Sequenzen der 3' und 5' nicht translatierten Regionen miteinschliessen.

Jeder Promotor und jeder Terminator, der in der Lage ist eine Induktion der Expression einer exprimierbaren DNA-Sequenz in Myxobakterien aus der Sorangium/Polyangium Gruppe zu bewirken, kann als Bestandteil der hybriden Genkonstruktion verwendet werden.

Promotoren, die für eine Verwendung in dem erfindungsgemässen Verfahren in Frage kommen, sind z.B.
- der durch Licht induzierbare Promotor von *Myxococcus xanthus* [EP 310 619];
- sonstige *Myxococcus xanthus* Promotoren;
- Promotoren von Actinomyceten, insbesondere von Streptomyceten;
- *E*. *coli* Promotoren, wie z.B. der Tac(hybrid)-, P_{L}- oder Trp-Promotor.

Geeignete Terminationssequenzen, die im Rahmen dieser Erfindung Anwendung finden können sind z.B. bei Rosenberg und Court (1979) sowie bei Gentz *et al* (1981) beschrieben.

Die somit gebildete funktionelle Einheit bestehend aus einem Gen und aus in den Myxobakterien-Zellen aktiven Expressionssignalen, kann anschliessend gegebenenfalls mit einem oder mehreren DNA-Abschnitten flankiert werden, die in einem Masse Homologien zu entsprechenden DNA-Regionen innerhalb des Myxobakteriengenoms aufweisen, dass bei der Transformation der die besagte homologe Region enthaltenden Myxobakterien-Zelle eine zufallsgemässe oder aber vorzugsweise eine gezielte Integration der von homologen DNA-Sequenzen flankierten Gensequenz an einer aufgrund der zwischen eingeschleuster DNA und bakterieneigener DNA vorhandenen Homologien definierten Stelle innerhalb des bakteriellen Genoms durch homologe Rekombination stattfindet. Die flankierenden, homologen DNA-Abschnitte liegen dabei im Rahmen dieser Erfindung vorzugsweise miteinander zu einer Einheit verschmolzen als Bestandteil eines ringförmig geschlossenen DNA-Moleküls vor.

In einer bevorzugten Ausführungsform wird dabei die einzuschleusende DNA in ein Plasmid integriert, das entweder bereits homologe DNA Abschnitte enthält oder aber diese zu einem späteren Zeitpunkt einkloniert erhält.

Sollen somit nicht nur einzelne Gene oder Genfragmente sondern die gesamte Plasmid-DNA, welche besagte Gene oder Genfragmente enthalten kann, in das Myxobakterien-Genom integriert werden, so ist es ausreichend, besagte homologe DNA-Sequenzen an einer beliebigen Stelle in die Plasmid-DNA einzuklonieren, wobei jedoch nach Möglichkeit die für eine Expression vorgesehenen Gene funktionell erhalten bleiben sollten. Auch in diesem Fall kann man somit die zu integrierende DNA (Plasmid-DNA) im Prinzip als von homologen DNA Sequenzen flankiert ansehen, da man sich diese homologen DNA Abschnitte innerhalb des ringförmig geschlossenen DNA Moleküls als miteinander zu einer Einheit verschmolzen denken kann.

Neben Strukturgenen können auch beliebige andere wünschenswerte Gene oder Genfragmente oder sonstige nützliche DNA-Sequenzen verwendet werden wie z.B. Bindestellen von Regulator-Molekülen, Promotoren, Terminator-Sequenzen, etc..

Durch die Wahl geeigneter DNA-Sequenzen homologen oder heterologen Ursprungs, die hinreichend grosse Homologien zu entsprechenden Abschnitten innerhalb des bakteriellen Genoms aufweisen und damit einen Austausch von genetischem Material via homologer Rekombination ermöglichen, ist es nunmehr erstmals möglich, Gene oder sonstige DNA-Sequenzen gezielt an vorherbestimmten Stellen des Myxobakteriengenoms zu integrieren und dort gegebenenfalls zu exprimieren.

Das Ausmass der für einen Austausch *via* homologer Rekombination benötigten Homologie zwischen den homolgen DNA-Abschnitten und der entsprechenden genomischen DNA-Region ist von verschiedenen Parametern abhängig und muss daher in Abhängigkeit von den verwendeten DNA-Sequenzen jeweils den entsprechenden Bedürfnissen angepasst werden. Man geht nach heutigem Kenntnisstand davon aus, dass eine homologe Region, die mindestens 100 Bp umfasst, ausreichend ist um das gewünschte Rekombinationsereignis herbeizuführen.

Bevorzugt im Rahmen dieser Erfindung ist eine homologe Region, die sich über einen Bereich von 0.3 bis 4 Kb, vorzugsweise aber über einen Bereich von 1 bis 3 Kb, erstreckt.

Für die Verwendung als homologe DNA-Abschnitte sind im Rahmen dieser Erfindung in erster Linie DNA-Sequenzen homologen Ursprungs geeignet, die durch Isolierung der Gesamt-Myxobakterien-DNA und anschliessender Verdauung mit geeigneten Restriktionsenzymen erhältlich sind.
Bei Kenntnis der DNA-Sequenz besagter homologer DNA-Fragmente können diese selbstverständlich auch synthetisch hergestellt werden.

Darüberhinaus können aber auch homologe DNA-Abschnitte heterologen Ursprungs verwendet werden, die nicht unmittelbar aus dem Genom des Zielorganismus isoliert wurden, sondern beispielsweise aus verwandten Organismen und die somit nicht notwendigerweise 100%ige Identität mit den entsprechenden DNA-Regionen auf dem Genom des Zielorganismus aufweisen, sondern diesen nur im wesentlichen homolog sind, d.h. einen Homologiegrad zwischen 80% und 100% und ganz besonders bevorzugt zwischen 90% und 100% aufweisen.

Einen wesentlichen Bestanteil der vorliegenden Erfindung bildet somit ein Verfahren zur gezielten genetischen Manipulation von Myxobakterien aus der Sorangium/Polyangium Gruppe das sich dadurch kennzeichnet, dass man genetisches Material homologen Ursprungs oder eine Kombination von genetischem Material homologen sowie heterologen Ursprungs in die Myxobakterienzelle einschleust und dort via homologer Rekombination gezielt an einer aufgrund der vorhandenen Homologien genau definierten Stelle in das Chromosom besagter Myxobakterien integriert.

Im Rahmen dieser Erfindung ist es somit nunmehr erstmals möglich, durch die Herstellung entsprechender hybrider Genkonstruktionen in der zuvor beschriebenen Weise gezielte Modifikationen von Bakterien-eigenen Genen innerhalb des Myxobakterien-genoms vorzunehmen oder aber zusätzliche Gene oder sonstige DNA-Fragmente in das Myxobakteriengenom einzubauen. Erfolgt die Intergration innerhalb eines funktionellen Gens oder Operons, so führt dies in der Regel zur Inaktivierung desselben und in der Folge zu einem phänotypisch beobachtbaren Defekt.

Im einzelnen kann man dabei so vorgehen, dass man die Myxobakterienzellen mit einem der zuvor beschriebenen rekombinanten DNA Moleküle transformiert, wobei die in besagtem rekombinanten DNA Molekül enthaltenen Gene, hybriden Genkonstruktionen oder sonstigen DNA-Fragmente durch homologe Rekombination zufallsgemäss oder aber vorzugsweise gezielt an einer aufgrund der vorhandenen Homologien definierten und somit vorherbestimmbaren Stelle in das bakterielle Genom integriert werden.

In einer spezifischen Ausführungsform der vorliegenden Erfindung erfolgt die Einschleusung des genetischen Materials in Myxobakterien aus der *SorangiumlPolyangium* Gruppe ungerichtet, über einen zu einem Konjugations ähnlichen Informations-Austausch mit dem Myxobakterium Zielorganismus befähigten Donororganismus von dem Donor auf den Myxobakterium-Rezipienten. Zur Herstellung geeigneter Plasmide, welche eine für die Integration *via* homologer Rekombination ausreichende Homologie mit dem Myxobakterien-Chromsom aufweisen, kann man beispielsweise so vorgehen, dass man zunächst die Gesamt-DNA von Myxobakterien isoliert und diese anschliessend fragmentiert. Diese Fragmentierung kann entweder mechanisch durch Einwirkung von Scherkräften oder aber vorzugsweise unter Verwendung geeigneter Restriktionsenzyme durchgeführt werden.

Aus der Vielzahl der anfallenden Fragmente können dann solche geeigneter Grösse isoliert und anschliessend in ein geeignetes Plasmid einkloniert werden.
Das Ligieren homologer DNA-Fragmente sowie von DNA Fragmenten homologen und heterologen Ursprungs in einen geeigneten Klonierungsvektor erfolgt mit Hilfe von Standardmethoden wie sie z.B. bei Maniatis *et al,* 1982 beschrieben sind.

Dabei wird in der Regel der Vektor und die zu integrierende DNA-Sequenz zunächst mit geeigneten Restriktions-Enzymen geschnitten. Geeignete Restriktionsenzyme sind z.B. solche, die Fragmente mit glatten Enden liefern, wie z.B. SmaI, HpaI und EcoRV, oder aber Enzyme, die kohesive Enden bilden, wie z.B. EcoRI, SacI, BamHI, SalI, PvuI, etc..

Sowohl Fragmente mit glatten Enden wie auch solche mit kohesiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der *E.coli* DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Deoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Grundsätzlich kann man für die Herstellung und Vervielfältigung der zuvor beschriebenen Konstrukte, die DNA Fragmente homologen oder aber eine Kombination von DNA Fragmenten homologen und heterologen Ursprungs enthalten, alle gängigen Klonierungsvektoren verwenden wie z.B. Plasmid- oder Bakteriophagen-Vektoren, sofern sie Replikations- und Kontrollsequenzen aufweisen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise, ohne das dies eine Limitierung des Erfindungsgegenstandes darstellt, Resistenzen gegen Ampicillin, Tetracyklin, Chloramphenicol, Hygromycin, G418, Kanamycin, Neomycin und Bleomycin. Als weiterer selektierbarer Marker kann beispielsweise eine Prototrophie für bestimmte Aminosäuren fungieren.

Bevorzugt im Rahmen der vorliegenden Erfindung sind in erster Linie *E*. *coli* Plasmide, wie z.B. das im Rahmen der vorliegenden Erfindung verwendete Plasmid pSUP2021.

Als Wirtszellen für die zuvor beschriebene Klonierung kommen im Rahmen dieser Erfindung in erster Linie Prokaryonten in Frage, einschliesslich bakterieller Wirte, wie z.B. *A*.*tumefaciens*, *E.coli*, *S.typhimurium* und *Serratia marcescens*, desweiteren Pseudomonaden, Actinomyceten, Salmonellen sowie Myxobakterien selbt.

Besonders bevorzugt sind *E*. *coli* Wirte, wie z.B. der *E*. *coli* Stamm HB101.

Kompetente Zellen des E. *coli* Stammes HB101 werden dabei mit Hilfe der üblicherweise für die Transformation von *E*. *coli* verwendeten Verfahren hergestellt [siehe: "Allgemeine rekombinante DNA Techniken"].

Nach Transformation und anschliessender Inkubation auf einem geeigneten Medium werden die resultierenden Kolonien einem diffentiellen Screening unterzogen durch Ausplattieren auf Selektivmedien. Anschliessend kann dann aus denjenigen Kolonien, welche Plasmide mit einklonierten DNA Fragmenten enthalten, die entsprechende Plasmid-DNA isoliert werden.

Auf diese Weise erhält man rekombinante Plasmide unterschiedlicher Grösse. Nach Restriktionsanalyse können dann Plasmide geeigneter Grösse für die sich anschliessende Einschleusung der Plasmid-DNA in die Myxobakterienzelle ausgewählt werden. Dieser DNA Transfer kann dabei entweder direkt erfolgen oder aber vorzugsweise über einen Zu einem konjugations ähnlichen Informations - Austausch mit dem Myxobakterinen Zielorganismus befähigten Zwischenwirt (Donorzelle) im Rahmen eines konjugalen Transfers.

Ein wesentlicher Bestandteil der vorliegenden Erfindung betrifft daher die Konstruktion von Plasmiden, die neben homologen Abschnitten auch eine oder mehrere Genkonstruktionen, bestehend aus einem oder mehreren Strukturgenen oder sonstigen wünschenswerten Genen bzw. Genfragmenten, die gegebenenfalls in operabler Weise mit in bakteriellen Zellen funktionsfähigen Expressionssignalen verknüpft sind, oder sonstige nützliche DNA-Sequenzen enthalten können. Die homologen DNA-Fragemente können dabei entweder gänzlich aus bakterieneigenen (Myxobakterien der Sorangium/Polyangium Gruppe) Genomabschnitten bestehen und somit vollständig homologen Ursprungs sein oder aber sie können neben homolgen Abschnitten auch mehr oder weniger ausgeprägte Anteile heterologen Ursprungs enthalten. Auch die Verwendung von homologen DNA-Abschnitten rein heterologen Ursprungs ist denkbar.

In einem weiteren Verfahrensschritt können diese Plasmide dazu verwendet werden, die zuvor beschriebene genetische Konstruktion, die gegebenenfalls ein Strukturgen enthält, welches für ein gewünschtes Genprodukt kodiert, in die Myxobakterienzelle einzuschleusen und dort in das Bakteriengenom zu integrieren.

Die Übertragung der anmeldungsgemässen genetischen Konstruktionen in die Myxobakterienzellen kann auf unterschiedliche Art und Weise erfolgen. Bevorzugt im Rahmen dieser Erfindung ist der konjugale Transfer von einer zu einem konjugationsähnlichen Informations-Austausch mit dem Myxobakterium Zielorganismus befähigten Donorzelle auf den Myxobakterien-Rezepienten.

Im Rahmen dieses konjugalen Transfers kann die zu transferrierende DNA dabei entweder zunächst, wie zuvor beschrieben, in einem der üblicherweise verwendeten Klonierungsvektoren kloniert und anschliessend in einen geeigneten Zwischenwirt transformiert werden, der als Donorzelle fungiert. Der Umweg über den Zwischenwirt kann vermieden werden, wenn man einen Wirtsstamm verwendet, der sowohl für die Klonierung von DNA als auch für den Einsatz als Donorzelle im Rahmen der Konjugation geeignet ist.

Zwischenwirte, die im Rahmen dieser Erfindung als Donorzellen Verwendung finden können sind im wesentlichen prokaryontische Zellen ausgewählt aus der Gruppe bestehend aus *E. coli*, Pseudomonaden, Actinomyceten, Salmonellen sowie Myxobakterien selbst.

Voraussetzung für den konjugalen Transfer von Plasmid-DNA von einer Donor-Zelle auf einen Rezepienten ist das Vorliegen von Transfer (tra)- und Mobilisierungsfunktionen (mob). Dabei muss die Mobilisierungsfunktion zumindest den Transfer-Ursprung (oriT) enthalten und auf dem zu transferierenden Plasmid liegen. Dagegen kann die Transfer-Funktion (tra) entweder auf dem Plasmid oder auf einem Helferplasmid lokalisiert sein oder aber integriert ins Chromosom der Donor-Zelle vorliegen.

Plasmide, welche die oben genannte Voraussetzung erfüllen und daher bevorzugt sind im Rahmen dieser Erfindung, fallen im wesentlichen in die Imkompabilitätsgruppen P, Q, T, N, W und ColI. Der Prototyp der P-Gruppen Plasmide ist das Plasmid RP4. Besonders bevorzugt im Rahmen dieser Erfindung ist das Plasmid pSUP2021, welches ein 1.9 Kb Fragment aus dem Plasmid RP4 enthält, das als Bestandteil der mob-Funktion (RP4mob) den Transfer-Ursprung (oriT) aufweist. Andere Plasmide mit der mob-Funktion (RP4mob), wie z.b. pSUP101, pSUP301, pSUP401, pSUP201, pSUP202, pSUP203 oder pSUP205 sowie die davon abgeleiteten Derivate [Simon *et al* (1988)] können ebenfalls im Rahmen des erfindungsgemässen Verfahrens verwendet werden.

Im Verlaufe der im Rahmen dieser Erfindung durchgeführten Experimente hat es sich gezeigt, dass es vorteilhaft ist, wenn man im Verlaufe des konjugalen Transfers den Myxobakterien-Rezipienten vor der Inkubation mit dem Donor-Stamm einer kurzfristigen Hitzebehandlung aussetzt. Bevorzugt ist eine ein bis 120 Minuten, insbesondere aber eine 5 bis 20 Minuten dauernde Vorinkubation der Rezipientenzellen bei einer Temperatur von 35°C bis 60°C, vorzugsweise bei einer Temperatur von 42°C bis 55°C und ganz besonders bevorzugt bei einer Temperatur von 48°C bis 52°C.

In einer bevorzugten Ausführungsform der vorliegenden Erfindung kommt ein *E*. *coli* Donor-Stamm zur Anwendung, der die Transfer-Gene (tra) von Plasmid RP4 eingebaut in die chromsomale DNA enthält. Bevorzugt im Rahmen dieser Erfindung ist der *E*. *coli* Donor Stamm W3101(pME305) der das Helferplasmid pME305 enthält, welches die Transfer-Funktion (tra) von RP4 besitzt.

Verfahrenstechnisch besonders interessant und damit besonders bevorzugt im Rahmen dieser Erfindung sind Bakterienstämme, die sowohl als Wirte für die Klonierung von Vektoren mit integrierten DNA-Sequenzen als auch für den Einsatz als Donorzelle im Rahmen des konjugalen Transfers geeignet sind. Ebenfalls besonders bevorzugt sind Bakterienstämme, die restriktionsnegativ sind, und somit eingeschleuste Fremd-DNA nicht abbauen. Beide der zuvor genannten Kriterien werden durch den *E. coli* Stamm ED8767(pUZ8) in idealer Weise erfüllt, der an dieser Stelle aber nur stellvertretend für andere geeignete Bakterienstämme genannt ist und die Anmeldung in keiner Weise limitieren soll.

Neben dem zuvor beschriebenen konjugalen Gentransfer von einer Donorzelle in einen Myxobakterien-Rezipienten, können selbstverständlich noch weitere geeignete Gentransfer-Verfahren zur Einschleusung von genetischem Material in Myxobakterien aus der Sorangium/Polyangium Gruppe Anwendung finden. Zu nennen wäre hier in erster Linie der Gentransfer *via* Elektroporation, im Rahmen dessen die Myxobakterienzellen kurzzeitig mit hohen elektrischen Feldstärken beaufschlagt werden [Kuspa und Kaiser (1989)]. Die allgemeinen Rahmenbedingungen für die Elektroporation von Prokaryontenzellen sind in US-P 4,910,140 im Detail beschrieben.

### NICHTLIMITIERENDE AUSFÜHRUNGSBEISPIELE

### Allgemeine rekombinante DNA-Techniken

Da viele der in dieser Erfindung verwendeten rekombinanten DNA-Techniken Routine sind für den Fachmann, soll im folgenden eine kurze Beschreibung dieser allgemein gebräuchlichen Techniken gegeben werden. Alle diese Verfahren in der Referenz von Maniatis *et al* (1982) beschrieben, es sei denn wird gesondert darauf hingewiesen.

### A. Schneiden mit Restriktionsendonukleasen

Typischerweise sind etwa 50 bis 500 µg/ml DNA in dem Reaktionsansatz enthalten, in der vom Hersteller, in erster Linie New England Biolabs, Beverly, MA. und Böhringer, Mannheim (BRD), empfohlenen Pufferlösung. 2 bis 5 Einheiten von Restriktionsendonukleasen werden für jedes µg DNA hinzugefügt und der Reaktionsansatz bei der vom Hersteller empfohlenen Temperatur für eine bis drei Stunden inkubiert. Die Reaktion wird durch 10 minütiges Erhitzen auf 65°C oder durch Extraktion mit Phenol, gefolgt durch Präzipitation der DNA mit Ethanol, beendet. Diese Technik wird auch auf den Seiten 104 bis 106 der Maniatis et al (1982)-Referenz beschrieben.

### B. Behandlung der DNA mit Polymerase, um glatte Enden zu erzeugen

50 bis 500 µg/ml DNA-Fragmente werden zu einem Reaktionsansatz hinzugefügt, in dem vom Hersteller, in erster Linie New England Biolabs, Beverly, MA. und Böhringer, Mannheim (BRD), empfohlenen Puffer.
Der Reaktionsansatz enthält alle vier Desoxynukleotidtriphosphate in Konzentrationen von 0.2 mM. Die Reaktion erfolgt während 30 Minuten bei 15°C und wird dann durch 10 minütiges Erhitzen auf 65°C beendet. Für Fragmente, die durch Schneiden mit Restriktionsendonukleasen erhalten werden, welche 5'-hervortretende Enden erzeugen, wie EcoRI und BamHI, wird das grosse Fragment, oder Klenow-Fragment, der DNA-Polymerase benutzt. Für Fragmente, die durch Endonukleasen erhalten werden, welche 3'-hervortretende Enden erzeugen, wie PstI und SacI, wird die T4-DNA-Polymerase benutzt. Die Verwendung dieser zwei Enzyme wird auf den Seiten 113 bis 121 der Maniatis e*t a*l (1982)-Referenz beschrieben.

### C. Agarose-Gelelektrophorese und Reinigung von DNA-Fragmenten von Gelen

Die Agarose-Gelelektrophorese wird in einem horizontalen Apparat durchgeführt, wie auf den Seiten 150 bis 163 der Maniatis et al.-Referenz beschrieben. Der benutzte Puffer ist der dort beschriebene Tris-Acetat Puffer. Die DNA-Fragmente werden durch 0.5 µg/ml Ethidiumbromid gefärbt, das entweder im Gel- oder Tankpuffer während der Elektrophorese vorhanden ist oder nach der Elektrophorese hinzugefügt wird. Die DNA wird durch Beleuchtung mit langwelligem Ultraviolettlicht sichtbar gemacht.

Wenn die Fragmente vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis *et al* (1982) auf Seite 170 beschriebenen leicht verändert.

Als Alternative kann die DNA aus der Agarose mit Hilfe des Geneclean Kits (Bio 101 Inc., La Jolla, CA, USA) isoliert werden.

### D. Hinzufügen von synthetischen Linkerfragmenten an DNA-Enden

Wenn es erwünscht ist, eine neue Endonukleaseschnittstelle an das Ende eines DNA-Moleküls anzufügen, wird das Molekül gegebenenfalls zuerst mit DNA-Polymerase behandelt, um glatte Enden zu erzeugen, wie im obigen Abschnitt beschrieben. Etwa 0.1 bis 1.0 µg dieses Fragments wird zu etwa 10 ng phosphorylierter Linker-DNA gegeben, die von New England Biolabs bezogen wurde, in einem Volumen von 20 bis 30 µl mit 2 µl T4 DNA-Ligase von New England Biolabs, und 1mM ATP in dem vom Hersteller empfohlenen Puffer.

Nach einer Inkubation über Nacht bei 15°C wird die Reaktion durch 10 minütiges Erhitzen auf 65°C beendet. Der Reaktionsansatz wird auf etwa 100 µl in einem Puffer verdünnt, der richtig für die Restriktionsendonuklease ist, die die synthetische Linkersequenz schneidet. Ungefähr 50 bis 200 Einheiten dieser Endonuklease werden hinzugefügt. Die Mischung wird 2 bis 6 Stunden bei der angemessenen Temperatur inkubiert, dann wird das Fragment einer Agarose-Gelelektrophorese unterworfen und gereinigt wie oben beschrieben. Das resultierende Fragment wird nun Enden haben mit Endigungen, welche durch Schneiden mit der Restriktionsendonuklease erzeugt wurden. Diese Enden sind gewöhnlich kohäsiv, so dass das resultierende Fragment nun leicht an andere Fragmente mit den gleichen kohäsiven Enden geknüpft werden kann.

### E. Entfernen von 5'-terminalen Phosphaten von DNA-Fragmenten

Während der Plasmidklonierungsschritte vermindert die Behandlung des Vektorplasmids mit Phosphatase die Rezirkularisation des Vektors (diskutiert auf Seite 13 der Maniatis e*t a*l-Referenz). Nach Schneiden der DNA mit der richtigen Restriktionsendonuklease wird eine Einheit alkalische Phosphatase aus dem Darm von Kälbern hinzugefügt, die von Boehringer-Mannheim, Mannheim, erworben wurde. Die DNA wird eine Stunde bei 37°C inkubiert und anschliessend zweimal mit Phenol extrahiert und mit Ethanol gefällt.

### F. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15°C durchgeführt. Wenn DNA-Fragmente mit glatten Enden verknüpft werden sollen, werden sie inkubiert wie oben, bis darauf, dass die Menge der T4 DNA-Ligase auf 2 bis 4 Einheiten erhöht wird.

### G. Die Transformation von DNA in E. coli

Die *E*. *coli* Stämme HB101, W3101 und ED8767 werden für die meisten Experimente verwendet. DNA wird mit dem Kalziumchloridverfahren, wie es von Maniatis *et al* (1982), Seiten 250 bis 251, beschrieben wurde, in E. c*o*l*i* eingeführt.

### H. Screening von E.coli auf Plasmide

Nach der Transformation werden die resultierenden Kolonien von *E*. *coli* auf das Vorhandensein des gewünschten Plasmids durch ein schnelles Plasmidisolationsverfahren geprüft. Zwei gebräuchliche Verfahren werden auf den Seiten 366 bis 369 der Maniatis *et al* (1982)-Referenz beschrieben.

### I. Isolierung von Plasmid-DNA in grossem Massstab

Verfahren zur Isolierung von Plasmiden aus *E.coli* in grossem Massstab werden auf den Seiten 88 bis 94 der Maniatis *et al* (1982)-Referenz beschrieben.

### Beispiele:

### Beispiel 1: Kultivierungsbedingungen für Sorangium

*Sorangium cellulosum* wird in einem G51b Flüssigmedium [siehe Abschnitt "Medien und Puffer] bei einer Temperatur von 30°C herangezogen. Die Kulturen werden durch Schütteln mit 180 Upm belüftet. Als Alternativmedium kann auch ein G52c Medium verwendet werden.

Für die Kultivierung auf Festmedium kann das im Abschnitt "Medien und Puffer" beschriebenen SolE Medium verwendet werden. Die Inkubationstemperatur beträgt auch in diesem Fall 30°C.

### Beispiel 2: Kultivierungsbedingungen für E. coli

*E*. *coli* Zellen werden in einem LB Medium [Miller (1972)] bei einer Temperatur von 37°C kultiviert.

### Beispiel 3: Herstellung einer Streptomycin-resistenten Spontanmutante von Sorangium cellulosum

200 µl einer drei Tage alten *Sorangium cellulosum* Kultur [Wildtyp-Stamm So ce 26], die in Flüssigmedium angezogen wurde, wird auf Festmedium [SolE-Medium] ausplattiert, das mit 300 µg/ml Streptomycin angereichert ist. Die Inkubationszeit beträgt 14 Tage bei einer Temperatur von 30°C. Bei den auf diesem Medium wachsenden Kolonien handelt es sich um spontane Streptomycin resistente Muatanten, die zur weiteren Konzentrierung und Reinigung nochmals auf dem gleichen Medium (mit Streptomycin) kultiviert werden.

Eine dieser Streptomycin resistenten Kolonien wird ausgewählt und erhält die Bezeichnung SJ3. Eine Probe dieses mutierten *Sorangium cellulosum* So ce 26 Stammes wurde am 25. 01. 1991 bei der gemäss den Bestimmungen des Budapester Vertrages als Internationale Hinterlegunsstelle annerkannten "Deutschen Sammlung von Mikroorganismen und Zellkulturen GmbH" [Braunschweig, BRD] unter der Hinterlegungsnummer DSM 6380 hinterlegt.

### Beispiel 4: Präparation der Sorangium Gesamt-DNA

Zur Isolierung der Gesamt-DNA wird eine in der stationären Phase befindliche *Sorangium* Kultur 10 Minuten bei 10'000 Upm zentrifugiert. Die abgetrennten Zellen werden dann in STE-Puffer [siehe Abschnitt "Medien und Puffer"] resuspendiert und auf eine Zelldichte von ca. 10⁹ Zellen/ml eingestellt.

Anschliessend werden 450 µl dieser Suspension mit 200 µl RLM-Puffer [siehe Abschnitt "Medien und Puffer"] und 2 µl Diethylpyrocarbonat vermischt. Um eine gründliche und gleichmässig Durchmischung zu gewährleisten bedient man sich geeigneter Geräte wie z.B. eines Vortex o.ä.. Nach 30 minütiger Inkubation bei 70°C in einem Inkubator werden 100 µl Kaliumacetat [5 M] zugegeben. Dieser Ansatz wird 15 Minuten auf Eis inkubiert und alle 5 Minuten gründlich durchmischt [Vortex]. Nach Zentrifugation [15 Minuten bei 10'000 Upm] wird der Überstand anschliessend zur Extraktion der Proteine mit 500 µl Phenol/Chloroform/Isoamylalkohol [25/24/1] vermischt. Nach erneuter Zentrifugation [15 Minuten bei 10'000 Upm] wird die obere Phase, welche die DNA Fraktion enthält, entnommen und der eventuell noch darin enthaltene Phenolanteil mit Diethylether [1 ml] entfernt. Anschliessend wird die DNA durch Zugabe von 1 ml Ethanol präzipitiert. Nach 30 minütiger Inkubation bei -70°C wird der gesamte Ansatz 15 Minuten bei 10'000 Upm zentrifugiert, das Pellet mit 70%igem Ethanol gewaschen und im Vakuum getrocknet. Zuletzt wird die DNA in TER-Puffer gelöst.

### Beispiel 5: Konjugativer Transfer von pSJB55 in Sorangiumcellulosum SJ3

### 5.1 Zweistufiges Verfahren

### 5.1.1 Einklonieren von Sorangium-DNA in Plasmid pSUP2021

Das aus dem *Sorangium* Stamm SJ3 isolierte Chromosom wird mit dem Restriktionsenzym PvuI geschnitten. Die auf diese Weise erhältlichen Fragmente werden in das Plasmid pSUP2021 [Simon R *et al* (1983]) einkloniert. Dabei werden 0.2 µg Plasmid DNA und 1 µg chromosomaler DNA zunächst mit PvuI verdaut und anschliessend mit Ethanol präzipitiert. Das Präzipitat wird abgetrennt, getrocknet und das getrocknete Pellet in 14 µl bidestilliertem Wasser resuspendiert. Danach werden 2.5 µl eines zehnfach konzentrierten Ligations-Puffers [siehe Abschnitt "Medien und Puffer"], 2.5 µl Rinderserumalbumin [0.1%], 2.5 µl ATP [10 mM], 2.5 µl DTT [0.2 M], 8 µl H₂O und 1 µl T4 DNA-Ligase zugegeben. Der gesamte Ligationsansatz wird dann über Nacht bei einer Temperatur von ca. 8°C inkubiert.

5 µl dieser Ligationsmischung werden zur Klonierung von rekombinanten Plasmiden in den *E*. *coli* Stamm HB101 transformiert. Hierzu werden kompetente Zellen des *E*. *coli* Stammes HB101 mit Hilfe der üblicherweise für die Transformation von *E*. *coli* verwendeten Verfahren hergestellt [siehe: "Allgemeine rekombinante DNA Techniken"].

Nach Transformation und anschliessender Inkubation während 24 Stunden auf mit Kanamycin [25 µg/ml] und Chloramphenicol [25 µg/ml] angereichertem LB-Agar werden die resultierenden Kolonien einem diffentiellen Screening unterzogen durch paralleles Ausplattieren auf Ampicillin-haltigem [60 µg/ml] und Ampicillin-freiem Medium. Anschliessend können diejenigen Kolonien isoliert werden, die aufgrund der Integration der Sorangium DNA-Fragmente ihre Ampicillinresistenz verloren haben. Von diesen Ampicillin-sensitiven Kolonien werden dann die Plasmide isoliert.

Auf diese Weise erhält man rekombinante Plasmide unterschiedlicher Grösse. Nach Restriktionsanalyse werden drei dieser Plasmide für die weiteren Experimente ausgewählt. Diese Plasmide mit der Bezeichnung pSJB50, pSJB55 und pSJB58 enthalten *Sorangium*-DNA Inserts von 1 Kb, 3.5 Kb bzw. 4 Kb.

### 5.1.2 Konjugativer Transfer von Plasmid pSJB55 in Sorangium cellulosum

Der Transfer von Plasmid pSJB55 in *Sorangium cellulosum* erfolgt unter Vermittlung des *E. coli* Stammes W3101(pME305) [Jaoua S *et al* (1987)], der zu einem konjugationsähnlichen Informationsaustausch mit *Sorangium* befähigt ist. Das E. *coli* Plasmid pME305 [Rella (1984)] dient dabei als Helferplasmid für die Mobilisierung von pSJB55.

Zunächst werden kompetente Zellen des *E. coli* Stammes W3101(pME305) mit Hilfe der üblicherweise für die Transformation von *E. coli* verwendeten Verfahren mit 5 µl der zuvor isolierten pSJB55 Plasmid-DNA transformiert. Die transformierten *E. coli* Zellen werden damit zum Donor für das Plasmid pSJB55.

Für den eigentlichen Transfer werden 15 ml einer in der stationären Phase befindlichen *Sorangium cellulosum* SJ3 Kultur [4 x 10⁸ Zellen/ml bis 1-4 x 10⁹ Zellen/ml] mit 10 ml einer späten Log-Phasen Kultur von *E. coli* Donor-Zellen, die einen vergleichbaren Anteil von Zellen enthalten, vermischt. Zusammen werden diese dann 10 Minuten bei 4000 bis 8000 Upm abzentrifugiert und in 500 µl eines G51b- oder G51t-Mediums resuspendiert.

Es hat sich als vorteilhaft erwiesen, wenn man die *Sorangium* Rezipienten-Zellen vor der Konjugation mit *E*.*coli* in einem Wasserbad kurzzeitig einer Hitzebehandlung aussetzt. Mit dem *Sorangium cellulosum* Stamm SJ3 lassen sich die besten Transfer-Ergebnisse mit einer 10 minütigen Hitzebehandlung bei einer Temperatur von 50°C erzielen. Unter diesen Bedingungen sind Transfer-Frequenzen von 1-5 x 10⁻⁵ erreichbar, was gegenüber einem Verfahren ohne vorherige Hitzebehandlung einer Steigerung um den Faktor 10 entspricht.

Nach Übertragung auf Platten mit So1E Festmedium erfolgt eine zweitägige Inkubation bei 30°C. Die Zellen werden dann geerntet und in 1 ml G51b- oder G51t-Medium resuspendiert. 100 µl dieser bakteriellen Suspension werden auf einem selektiven So1E-Medium ausplattiert, das neben Kanamycin [25 mg/l] noch Phleomycin [20 bis 35 mg/l] und Streptomycin [300 mg/l] als selektive Agentien enthält. Die Gegenselektion des Donor-Stammes [*E*. *coli* W3101(pME305)] erfolgt mit Hilfe von Streptomycin.

Bei den auf diesem selektiven So1E Medium nach einer Inkubationszeit von 10 bis 14 Tagen gewachsenen Kolonien handelt es sich um Transkonjuganten von *Sorangium cellulosum*, die durch konjugativen Transfer des Plasmids pSJB55 eine Phleomycinresistenz erworben haben. Diese Phleomycin-resistenten Kolonien können für die weiteren molekularbiologischen Untersuchungen verwendet werden. Die Transformationsfrequenz für den Transfer von Plasmid pSJB55 auf *Sorangium* liegt bei durchschnittlich 3 x 10⁻⁶ bezogen auf den Rezipienten-Stamm SJ3.

Die Plasmide pSJB 50 und pSJB58 können in analoger Weise auf Sorangium transferriert werden.

### 5.2 Einstufiges Verfahren

### 5.2.1 Einklonieren von Sorangium-DNA in Plasmid pSUP2021

Das Einklonieren von *Sorangium*-DNA in Plasmid pSUP2021 kann wie in Beispiel 5.1.1 beschrieben durchgeführt werden.

Durch den Austausch des in 5.1.1 verwendeten Helferplasmids pME305 gegen das Plasmid pUZ8 [Hedges and Matthew (1979)], das im Gegensatz zu dem zuvor genannten Plasmid kein Ampicillin-Resistenzgen trägt, kann der Klonierungsschritt im *E.coli* Zwischenwirt HB101 entfallen, da jetzt eine direkte Klonierung in dem für den konjugalen Transfer vorgesehenen *E.coli* Donorstamm ED8767 möglich ist.

Bei dem Plasmid pUZ8 handelt es sich um einen Abkömmling des einen weiten Wirtsbereich umfassenden Plasmids RP4, das bei Datta *et al* (1971) beschrieben ist. Die Modifikationen gegenüber dem Ausgangsplasmid RP4 betreffen im wesentlichen das Ampicillin-Resistenzgen sowie das Insertionselement IS21, die beide deletiert sind sowie den Einbau eines zusätzlichen Gens, das eine Resistenz gegenüber Quecksilberionen vermittelt [siehe Jaoua *et al* (1987)].

Die gemäss Beispiel 5.1.1 hergestellte Ligationsmischung kann daher jetzt direkt in den *E. coli* Stamm ED8767 transformiert werden. Hierzu werden kompetente Zellen des *E. coli* Stammes ED8767 mit Hilfe der üblicherweise für die Transformation von *E. coli* verwendeten Verfahren hergestellt [siehe: "Allgemeine rekombinante DNA Techniken"].

Nach Transformation und anschliessender Inkubation während 24 Stunden auf mit Tetracyclin [10 µg/ml] und Chloramphenicol [25 µg/ml] angereichertem LB-Agar werden die resultierenden Kolonien einem diffentiellen Screening unterzogen durch paralleles Ausplattieren auf Ampicillin-haltigem [60 µg/ml] und Ampicillin-freiem Medium. Anschliessend können diejenigen Kolonien isoliert werden, die aufgrund der Integration der *Sorangium* DNA-Fragmente ihre Ampicillinresistenz verloren haben. Die so erhältlichen Kulturen können dann direkt als Donorzellen für den konjugativen Transfer der rekombinanten Plasmide in *Sorangium cellulosum* Zellen eingesetzt werden.

Anstelle der oben erwähnten Ligationsmischung können hier selbstverständlich auch die gemäss Beispiel 5.1.1 hergestellten rekombinanten Plasmide pSJB50, pSJB55 oder pSJB58 in den *E.coli* Stamm ED 8767 einkloniert werden.

### 5.2.2 Konjugativer Transfer der rekombinanten Plasmide in Sorangium cellulosum

Für den eigentlichen Transfer werden 15 ml einer in der stationären Phase befindlichen *Sorangium cellulosum* SJ3 Kultur [1-4 x 10⁹ Zellen/ml] mit 10 ml einer späten Log-Phasen Kultur von *E*. *coli* ED8767 Donor-Zellen, die einen vergleichbaren Anteil von Zellen enthalten, vermischt. Zusammen werden diese dann 10 Minuten bei 4000 Upm abzentrifugiert und in 500 µl eines G51b- oder G51t-Mediums resuspendiert.

Auch in diesem Fall erweist es sich als vorteilhaft, wenn man die *Sorangium* Rezipienten-Zellen vor der Konjugation mit *E*.*coli* in einem Wasserbad kurzzeitig einer Hitzebehandlung aussetzt. Mit dem *Sorangium cellulosum* Stamm SJ3 lassen sich die besten Transfer-Ergebnisse mit einer 10 minütigen Hitzebehandlung bei einer Temperatur von 50°C erzielen. Unter diesen Bedingungen sind Transfer-Frequenzen von 1-5 x 10⁻⁵ erreichbar, was gegenüber einem Verfahren ohne vorherige Hitzebehandlung einer Steigerung um den Faktor 10 entspricht.

Die weitere Kultivierung der transformierten *Sorangium* Zellen erfolgt analog zu der in Beispiel 5.1.2 beschriebenen Vorgehensweise.

Durch die Verwendung eines Restriktions-negativen *E.coli* Stammes als Donor-Stamm, wie z.B. *E.coli* ED8767 [Murray *et al* (1977)] kann die Transformationsfrequenz im Unterschied zu dem zuvor beschriebenen Verfahren drastisch erhöht werden (bis zu einem Faktor 10³).

### Molekulargenetische Analyse

### (A) Nachweis der Integration des Plasmids pSJB55 in das Chromosom von Sorangium cellulosum SJ3

Die aus den transformierten *Sorangium* Zellen gemäss obiger Beschreibung [vergl. Beispiel 4] isolierte Gesamt-DNA wird mit SmaI bzw SalI verdaut und auf ein horizontales Tris-acetat [40 mM Tris-HCl, 20 mM Natriumacetat, 2 mM EDTANa2, pH 7.8] Agarosegel [0.9%] aufgetragen. Nach erfolgter Elektrophorese wird das Gel zunächst für 30 Minuten in eine denaturierende Lösung [1.5 M NaCl, 0.5 M NaOH] und anschliessend in eine neutralisierende Lösung [1.5 M NaCl, 0.5 M Tris-HCl, 1 mM EDTANa2, pH 7.2] eingebracht. Mittels eines 'Southern Capillary Blottings' wird die DNA dann unter Verwendung eines 20fach konzentrierten SSC-Puffers [siehe Abschnitt "Medien und Puffer"] auf eine Nylonmembrane [z.B. eine "Amersham Hybond nylon membrane"; Amersham International plc, Amersham Place, Amersham, England HP7 9NA] transferriert und dort durch 6 minütige UV-Behandlung fixiert. Weitere Einzelheiten dieses Verfahrens sind im Handbuch von Amersham International, "Membran Transfer and Detection Methods", (1985) beschrieben.

Die als Hybridisierungsprobe vorgesehene DNA wird mittels einer Nicktranslation markiert [Rigby DWJ *et al* (1977)]. Es handelt sich dabei um das ³²P-markierte, 3.5 Kb umfassende PvuI Insert aus Plasmid pSJB55. Die eigentliche Hybridisierung wird unter Verwendung eines leicht modifizierten Verfahrens nach Denhardt [Denhardt DT (1976)] durchgeführt. Der für die Prähybridisierung und Hybridisierung verwendete Puffer weist folgende Zusammensetzung auf: 6 x SSC [Maniatis *et al* (1982)] + 5 x Denhard [Maniatis *et al* (1982)] + 0.5% SDS + 0.2 mg/ml denaturierte "salmon sperm" DNA.

Die Prähybridisierung wird bei 65°C durchgeführt und dauert 3 Stunden, während die eigentliche Hybridisierungsreaktion nach 20 Stunden abgeschlossen ist. Zur Hybridisierung wird ein ³²P-markiertes [10⁵ cpm pro cm² Filter], denaturiertes, 3,5 Kb umfassendes PvuI Fragment von Plasmid pSJB55 zugegeben. Nach der Hybridisierung wird der Filter zunächst 2 x 15 Minuten in 2fach konzentriertem SSC bei einer Temperatur von 65°C gewaschen, anschliessend 30 Minuten in 2 x SSC + 0.1% SDS ebenfalls bei 65°C und schliesslich ein weiters mal in 0.5 x SSC [15 Minuten bei 65°C].

Die sich anschliessende Autoradiographie wird unter Verwendung eines Röntgenfilms [z.B. FUJY X Rays film] durchgeführt. Die Autoradiogramme der Transkonjuganten zeigen keine Banden, die mit freier, in überspriralisierter Form vorliegender pSJB55 Plasmid-DNA korrespondieren. Dagegen findet man aber ein positives Signal in der chromosomalen Region der Filtermembran.

Das SmaI verdaute Plasmid pSJB55 liefert 3 Banden von 8.9, 6.7 und 1.6 Kb. Das Hybridisierungsmuster des Elternstammes SJ3 zeigt nach SmaI Verdauung ebenfalls drei Banden, wovon eine dem internen, 1.6 Kb umfassenden Fragment des in Plasmid pSJB55 einklonierten *Sorangium* Inserts entspricht. Das Hybridisierungsmuster der SmaI verdauten DNA der Transkonjuganten zeigt 5 Banden [8.9 und 6.7 Kb Bande von Plasmid pSJB55 sowie SJ3 Banden] einschliesslich der allen drei gemeinsamen 1.6 Kb Bande.

Nach SalI Verdauung findet man für Plasmid pSJB55 eine Bande von 14.1 Kb (das andere 3.1 Kb umfassende SalI-Fragment von pSJB55 hybridisiert nicht mit der Probe). Das Hybridisierungsmuster der SalI verdauten SJ3 DNA zeigt ebenfall eine Bande von 5 Kb. Bei den Transkonjuganten verschwindet das 14.1 Kb Fragment von Plasmid pSJB55 sowie das 5 Kb Fragment von SJ3 nach SalI Verdauung. Diese werden ersetzt durch zwei neue Banden von 11.5 Kb und 7.7 Kb.

Die SmaI Daten zeigen, dass sämliche pSJB55 Fragmente intakt im Genom der Transkonjuganten vorliegen. Damit ist die Möglichkeit einer ortsspezifischen Rekombination ausgeschlossen, da in diesem Fall zumindest eines der SmaI Fragmente hätte verschwinden müssen.
Die Ergebnisse der SalI Verdauung machen darüberhinaus deutlich, dass das Plasmid pSJB55 in das *Sorangium* Genom integriert wurde und zwar an der Stelle, wo der zu pSJB55 homologe DNA Bereich sich befindet (=3.5 Kb PvuI Fragment). Diese Integration findet im Zuge einer homologen Rekombination zwischen dem 3.5 Kb umfassenden *Sorangium* Insert aus pSJB55 und dem gleichen Insert innerhalb des *Sorangium* Genoms statt.

### MEDIEN UND PUFFERLÖSUNGEN

| G51b-Medium (pH 7.4) | |
|---|---|
| Glucose | 0.2 % |
| Stärke | 0.5 % |
| [Kartoffelstärke, Typ Noredux; CERESTAR ITALIA S.p.a., Mailand, Italien] | |
| Pepton [DIFCO Laboratories, USA] | 0.2 % |
| Probion S | 0.1% |
| [Single Cell Protein; HÖCHST AG, Frankfurt, BRD] | |
| CaCl₂ x 2 H₂O | 0.05% |
| MgSO₄ x 7 H₂O | 0.05% |
| HEPES [FLUKA] | 1.2 % |

| G51t-Medium (pH 7.4) | |
|---|---|
| Glucose | 0.2 % |
| Stärke | 0.5 % |
| [Kartoffelstärke, Typ Noredux; CERESTAR ITALIA S.p.a., Mailand, Italien] | |
| Trypton [MARCO, Hackensack, NJ USA] | 0.2 % |
| Probion S | 0.1 % |
| [Single Cell Protein; HÖCHST AG, Frankfurt, BRD] | |
| CaCl₂ x 2 H₂O | 0.05% |
| MgSO₄ x 7 H₂O | 0.05% |
| HEPES [FLUKA] | 1.2 % |

| G52c-Medium (pH 7.4) | |
|---|---|
| Glucose | 2.0 g/l |
| Stärke | 8.0 g/l |
| [Kartoffelstärke, Typ Noredux; CERESTAR ITALIA S.p.a., Mailand, Italien] | |
| Sojamehl entfettet | 2.0 g/l |
| [MUCEDOLA S.r.l., Settimo Milanese, Italien] | |
| Hefeextrakt | 2.0 g/l |
| [FOULD & SPRINGER, Maison Alfort, Frankreich] | |
| CaCl₂ x 2 H₂O | 1.0 g/l |
| MgSO₄ x 7 H₂O | 1.0 g/l |
| Fe-EDTA [8 g/l Stammlösung] | 1.0 ml |
| HEPES [FLUKA] | 2.0 g/l |
| Dest. Wasser ad | 1000 ml |

pH-Wert wird vor der Sterilisation [20 Minuten bei 120°C] mit NaOH auf 7.4 eingestellt. pH nach Sterilisation: 7.4

| So1E-Medium (pH 7.4) | |
|---|---|
| Glucose* | 0.35 % |
| Trypton [MARCO, Hackensack, NJ USA] | 0.05 % |
| MgSO₄ x 7 H₂O | 0.15 % |
| Ammoniumsulfat* | 0.05 % |
| CaCl₂ x 2H₂O* | 0.1 % |
| K₂HPO₄^{*} | 0.006 % |
| Natriumdithionit* | 0.01 % |
| Fe-EDTA* | 0.0008% |
| HEPES [FLUKA] | 1.2 % |
| Überstand einer sterilisierten, | |
| stationären *S*. *cellulosum* Kultur* | 3.5 % (v/v) |
| Agar | 1.5 % |
| *Zugabe erfolgt erst nach Sterilisation | |
| pH-Wert wird vor der Sterilisation [20 Minuten bei 120°C] mit NaOH auf 7.4 eingestellt. | |

| LB-Medium | |
|---|---|
| Trypton | 10.0 g/l |
| Hefeextrakt | 5.0 g/l |
| NaCl | 5.0 g/l |

| STE-Puffer (pH 8.0) | |
|---|---|
| Saccharose | 25 % |
| EDTANa2 | 1 mM |
| Tris-HCl | 10 mM |

| RLM-Puffer (pH 7.6) | |
|---|---|
| SDS | 5 % |
| EDTANa2 | 125 mM |
| Tris-HCl | 0.5 mM |

| TER-Puffer | |
|---|---|
| Tris-HCl (pH 8.0) | 10 mM |
| 1 mM EDTANa2 | 1 mM |
| RNAse | 10 µg/ml |

| Ligations-Puffer | |
|---|---|
| MgCl₂ | 0.1 M |
| Tris-HCl (pH 7.8) | 0.5 M |

### TABELLEN

**Tabelle 1:**

| Bakterienstämme und Plasmide | |
|---|---|
| Stamm | Relevante Charakterisika |
| ***Escherichia coli*** | |
| W3101Na1 | RecA13, trpE, Na1R |
| HB101 | F-, hsds20 (r-, m-), recAl3, ara 14, proA2, lacY1, galK2, rpsL20 (SmR), xyl-5, mtl-1, sup E 44, lambda- |
| ED8767 | recA, supE, supF, hsdS |

| ***Sorangium cellulosum*** | |
|---|---|
| | |
| So ce 26 | Wildtyp Stamm |
| So ce 26/SJ3 | SmR Spontanmutante |
| | |

| Plasmid | |
|---|---|
| | |
| pSUP2021 | Ap, Cm, Km, Ph |
| pSJB50 | Cm, Km, Ph |
| pSJB55 | Cm, Km, Ph |
| pSJB58 | Cm, Km, Ph |
| pME305 | Ap, Tc |
| pUZ8 | Tc, Km, Hg |

### HINTERLEGUNG

Im Rahmen der vorliegenden Anmeldung wurden die folgenden Mikroorganismen bzw. Plasmide bei der gemäss Budapester Vertrag als Internationale Hinterlegungstelle anerkannten "Deutschen Sammlung von Mikroorganismen und Zellkulturen GmbH" in Braunschweig (BRD) entsprechend den Anforderungen für die Internationale Anerkennung der Hinterlegung von Mikroorganismen zum Zwecke der Patentierung hinterlegt.

| ***Mikroorganismus/ Plasmid*** | ***Hinterlegungs- datum*** | ***Hinterlegungs- nummer*** | ***Datum der Lebens- fähigkeitsbeschei- nigung*** |
|---|---|---|---|
| pSJB55 (einkloniert in *E.coli*) | 25.01.1991 | DSM 6321 | 25.01.1991 |
| *Sorangium cellulosum* So ce 26/SJ3 | 25.01.1991 | DSM 6380 | 14.02.1991 |

### LITERATURVERZEICHNIS

**Breton** AM *et al*, J Bacteriol, 161: 523-528 (1985)
**Breton** AM *et al*, *J* Biotechnol, 4: 303-311 (1986)
**Breton** AM und Guespin-Michel JF, FEMS Microbiol Lett, 40: 183-188 (1987)
**Datta** N *et al*, J Bacteriol 108: 1244-1249 (1971)
**Denhardt** DT, Biochem Biophys Res Commun, 23: 641-646 (1976)
**Hedges** RW und Matthew M, Plasmid 2: 269-278 (1979)
**Gentz** R *et al*, Proc Natl Acad Sci, USA 78: 4926-4940 (1981)
**Jaoua** S *et al*, Plasmid 18: 111-119 (1987)
**Jaoua** S *et al*, Plasmid 23: 183-193 (1990)
**Kaiser** D, Genetics of Myxobacteria, in: "Myxobacteria: Development and Cell Interactions", ed E Rosenberg, pp 163-184, Springer Verlag, Berlin/New York (1984);
**Kuner** JM und Kaiser D, Proc Natl Acad Sci USA, 78: 425-429 (1981)
**Kuspa A** und Kaiser D, J Bacteriol 171: 2762-2772 (1989)
**Maniatis** T e*t a*l, "Molecular Cloning", Cold Spring Harbor Laboratory, Cold Spring Harbor, NY (1982)
**Miller** JH, "Experiments in Molecular Genetics", Cold Spring Harbor Laboratory, Cold Spring Harbor, NY (1972)
**Murray** NE *et al*, Mol Gen Genet 150: 53 (1977)
**O'Conner** KA und Zusman DR, J Bacteriol, 155: 317-329 (1983)
**Rella** M, Dissertation ETH Zürich, No. 7601.SFITZ
**Reichenbach** H *et al*, Trends in Biotechnology, 6: 115-121 (1988)
**Rigby** DWJ *et al*, J Mol Biol, 113: 237-251 (1977)
**Rosenberg** M und Court D, Ann Rev Genetics 13: 319-353 (1979)
**Shimkets** LJ und Asher SY Mol Gen Genet 211: 63-71 (1988)
**Shimkets** LJ *et al*, Proc Natl Acad Sci USA, 80: 1406-1410 (1983)
**Simon** R *et al*, Bio/Technol, November 1983: 784-791 (1983)

**EP 0 310 619**
**EP 0 358 606**
EP 0 372 230
**US-P 4,910,140**

## Patentansprüche

1. Verfahren zur genetischen Manipulation von **Myxobakterien** aus der ***Sorangium/Polyangium* Gruppe**, dadurch kennzeichnet, dass man
(a) genetisches Material homologen oder heterologen Ursprungs oder eine Kombination von genetischem Material homologen sowie heterologen Ursprungs, **das** zu einem korrespondierenden Bereich auf dem Myxobakterienchromosom homolog oder aber zumindest im wesentlichen homolog ist; oder aber
(b) genetisches Material, welches natürlicherweise keine zu einem korrespondierenden Bereich auf dem Myxobakterienchromosom homologen oder aber zumindest im wesentlichen homologen Abschnitte enthält und daher artifiziell mit Hilfe an sich bekannter rDNA Techniken mit solchen homologen oder aber im wesentlichen homologen DNA Abschnitten verknüpft wird, **wodurch das einzuschleusende genetische Material von besagten homologen oder aber im wesentlichen homologen DNA Abschnitten flankiert wird;**
in die Myxobakterienzelle einschleust und dort via homologer Rekombination an einer aufgrund der zwischen eingeschleuster DNA und bakterieneigener DNA vorhandenen Homologien genau definierten Stelle in das Chromosom besagter Myxobakterien integriert.

2. Verfahren gemäss Anspruch 1, dadurch gekennzeichnet, dass es sich bei besagtem einzuschleusendem genetischem Material um eine exprimierbare DNA handelt, die in operabler Weise mit in der Myxobakterienzelle funktionsfähigen Expressions-Sequenzen verknüpft ist.

3. Verfahren gemäss Anspruch 1, dadurch gekennzeichnet, dass die Einschleusung des genetischen Materials über einen **zu einem konjugationsähnlichen Informations-Austausch mit dem Myxobaketerium Zielorganismus befähigten Donororganismus** von **dem** Donor auf den Myxobakterium-Rezipienten erfolgt.

4. Verfahren zur genetischen Manipulation von **Myxobakterien aus der *Sorangium/Polyangium* Gruppe** gemäss Anspruch 1, das durch die folgenden Verfahrensschritte gekennzeichnet ist:
(a) Präparation der Gesamt-DNA des **Myxobakterium** Zielorganismus oder eines verwandten Organismus;
(b) Fragmentieren der gemäss (a) isolierten Gesamt-DNA;
(c) Einklonieren der gemäss (b) hergestellten Fragmente in geeignete Plasmid-Vektoren und Transformation besagter Vektoren in einen der üblicherweise für Klonierungszwecke verwendeten Wirtsorganismen;
(d) Selektion derjenigen Kolonien, die ein Plasmid mit integriertem **Myxobakterium**-DNA-Fragment enthalten und Isolierung besagter Plasmide;
(e) Transformation der gemäss (d) isolierten Plasmid-DNA in einen zu einem konjugationsähnlichen Informations-Austausch mit dem **Myxobakterium** Zielorganismus befähigten Donororganismus;
(f) Konjugaler Transfer der rekombinanten Plasmid DNA auf den **Myxobakterium** Zielorganismus;
(g) Kultivierung der transformierten **Myxobakterium** Zellen und Selektion positiver Transformanten.

5. Verfahren gemäss einem der Ansprüche 1 oder **4,** dadurch gekennzeichnet, dass es sich bei dem einzuschleusenden genetischen Material um ein Plasmid handelt, das gegebenenfalls eine oder mehrere exprimierbare DNA-Abschnitte enthält und das die homologen oder aber die im wesentlichen homologen DNA Bereiche einkloniert enthält.

6. Verfahren gemäss einem der Ansprüche 1 oder **4**, dadurch gekennzeichnet, dass der Grad der Homologie zwischen besagten homologen DNA Abschnitten und den korrespondierenden Bereichen innerhalb des Myxobakterienchromosoms zwischen **80**% und 100% beträgt.

7. Verfahren gemäss einem der Ansprüche 1 oder **4**, dadurch gekennzeichnet, dass besagte homologe DNA Abschnitte wenigstens 100 Bp umfassen.

8. Verfahren gemäss einem der Ansprüche 1 oder **4**, dadurch gekennzeichnet, dass besagte homologe DNA Abschnitte zwischen 0,3 und 4 Kb umfassen.

9. Verfahren gemäss Anspruch **4**, dadurch gekennzeichnet, dass man als Wirtsorganismus für die Klonierung der **Myxobakterium**-DNA-Fragmente bzw als Donororganismus im Rahmen des konjugalen DNA-Transfers einen Prokaryonten ausgwählt aus der Gruppe bestehend aus *E. coli*, Pseudomonaden, Actinomyceten, Salmonellen sowie Myxobakterien selbst verwendet.

10. Verfahren gemäss Anspruch **4**, dadurch gekennzeichnet, dass man als Wirtsorganismus für die Klonierung der **Myxobakterium**-DNA-Fragmente bzw als Donororganismus im Rahmen des konjugalen DNA-Transfers einen restriktionsnegativen oder restriktionsdefizienten Bakterienstamm verwendet.

11. Verfahren gemäss Anspruch **10**, dadurch gekennzeichnet, dass man die Klonierung der **Myxobakterium**-DNA-Fragmente direkt in einem zu einem konjugationsähnlichen Informations-Austausch mit dem **Myxobakterium** Zielorganismus befähigten Donororganismus durchführt.

12. Verfahren gemäss Anspruch **4**, dadurch gekennzeichnet, dass man die **Myxobakterium**-Zellen unmittelbar vor dem konjugalen DNA Transfer einer kurzzeitigen Hitzebehandlung unterzieht.

13. Verfahren gemäss Anspruch **12**, dadurch gekennzeichnet, dass die Hitzebehandlung über einen Zeitraum von 1 und 120 Minuten bei einer Temperatur von 35°C bis 60°C durchgeführt wird.

14. Rekombinantes DNA Molekül, welches eine Integration von genetischem Material an einer definierten Stelle innerhalb des Genoms von Myxobakterien **aus der *Sorangium/Polyangium* Gruppe** ermöglicht, dadurch gekennzeichnet, dass besagtes rekombinantes DNA Molekül die zu integrierende DNA enthält, und dass besagte DNA in einem Masse Homologien zu entsprechenden DNA Regionen innerhalb des myxobakteriellen Genoms aufweist oder aber von einer oder mehreren solchen homologen DNA-Sequenzen flankiert wird, dass bei der Transformation der die homologen DNA Regionen enthaltenden Myxobakterien-Zelle eine Integration besagter zu integrierender DNA an einer aufgrund der zwischen eingeschleuster DNA und bakterieneigener DNA vorhandenen Homologien genau definierten Stelle innerhalb des myxobakteriellen Genoms via homologer Rekombination stattfindet.

15. Rekombinantes DNA Molekül gemäss Anspruch **14,** dadurch gekennzeichnet, dass der Grad der Homologie zwischen besagten homologen DNA Abschnitten und den korrespondierenden Bereichen innerhalb des Myxobakterienschromosoms zwischen **80** und 100% beträgt.

16. Rekombinantes DNA Molekül gemäss Anspruch **14,** dadurch gekennzeichnet, dass besagte homologen DNA Abschnitte einen Bereich von wenigstens 100 Bp umfassen.

17. Rekombinantes DNA Molekül gemäss Anspruch **14**, dadurch gekennzeichnet, dass besagte zu integrierende DNA-Sequenz bereits selbst eine genügend grosse Homologie zu entsprechenden DNA Regionen innerhalb des bakteriellen Genoms aufweist, so dass ein direkter Austausch dieser DNA-Sequenz gegen besagte homologe genomische DNA vermittels der homologen Rekombination erfolgen kann.

18. Rekombinantes DNA Molekül gemäss Anspruch **14**, dadurch gekennzeichnet, dass es sich bei besagter einzuschleusender DNA um doppelsträngige DNA handelt.

19. Rekombinantes DNA Molekül gemäss Anspruch **14**, dadurch gekennzeichnet, dass es sich bei besagter einzuschleusender DNA um einzelsträngige DNA handelt.

20. Rekombinantes DNA Molekül gemäss Anspruch **14**, dadurch gekennzeichnet, dass es sich bei besagter einzuschleusender DNA um eine exprimierbare DNA handelt, die in operabler Weise mit in der Myxobakterienzelle funktionsfähigen Expressions-Sequenzen verknüpft ist.

21. Rekombinantes DNA Molekül gemäss Anspruch **14,** dadurch gekennzeichnet, dass besagte flankierende DNA Abschnitte miteinander zu einer Einheit verschmolzen als Bestandteil eines ringförmig geschlossenen DNA Moleküls vorliegen.

22. Rekombinantes DNA Molekül gemäss Anspruch **14,** dadurch gekennzeichnet, dass besagte homologen DNA Abschnitte aus dem Myxobakterium-Genom selbst stammen.

23. Klonierungsvektor enthaltend ein rekombinantes DNA Molekül gemäss einem der Ansprüche **14** bis **22.**

24. Plasmid DNA für den konjugalen Transfer von einem Donororganismus auf den **Myxobakterium**-Rezipienten **aus der *Sorangium/Polyangium* Gruppe,** dadurch gekennzeichnet, dass besagte Plasmid DNA neben der einzuschleusenden DNA homologe oder aber im wesentlichen homologe DNA Abschnitte sowie für einen Transfer in **Myxobakterium**-Zellen geeignete Transfer (tra)- und Mobilisierungsfunktionen (mob) enthält.

25. Plasmid DNA gemäss Anspruch **24** enthaltend ein rekombinantes DNA Molekül gemäss einem der Ansprüche **14** bis **22**.

26. Verfahren zur Herstellung eines rekombinanten DNA Moleküls gemäss Anspruch **14,** dadurch gekennzeichnet, dass man die zu integrierende DNA, die in einem Masse Homologien zu entsprechenden DNA-Regionen innerhalb des myxobakteriellen Genoms aufweist, dass bei der Transformation des die besagte homologen Region enthaltenden Myxobakterien-Genoms eine Integration besagter DNA an einer aufgrund der zwischen eingeschleuster DNA und bakterieneigener DNA vorhandenen Homologien definierten Stelle innerhalb des bakteriellen Genoms via homologer Rekombination stattfindet.
(a) aus einer Quelle isoliert, **die homologe oder aber im wesentlichen homologe DNA Abschnitte enthält**; oder
(b) sofern besagte zu integrierende DNA natürlicherweise keine zu einem korrespondierenden Bereich auf dem Myxobakterienchromosom homologen oder aber zumindest im wesentlichen homologen Abschnitte enthält, diese artifiziell mit Hilfe an sich bekannter rDNA Techniken mit entsprechenden homologen oder aber im wesentlichen homologen DNA-Abschnitten verknüpft.

27. Verfahren gemäss Anspruch **26,** dadurch gekennzeichnet, dass sich besagte einzuschleusende DNA auf einem Plasmid befindet und die homologen DNA-Sequenzen an einer beliebigen Stelle in die Plasmid-DNA einkloniert werden, ohne dabei jedoch die funktionelle Integrität der einzuschleusenden DNA zu zerstören.

28. Verfahren zur Herstellung genetisch modifizierter **Myxobakterien aus der *Sorangium/Polyangium* Gruppe gemäss Anspruch 1**, dadurch kennzeichnet, dass man
(a₁) genetisches Material homologen oder heterologen Ursprungs oder eine Kombination von genetischem Material homologen sowie heterologen Ursprungs, **das** zu einem korrespondierenden Bereich auf dem Myxobakterienchromosom homolog oder aber zumindest im wesentlichen homolog **ist**; oder aber
(a₂) genetisches Material, welches natürlicherweise keine zu einem korrespondierenden Bereich auf dem Myxobakterienchromosom homologen oder aber zumindest im wesentlichen homologen Abschnitte enthält und daher artifiziell mit Hilfe an sich bekannter rDNA Techniken mit solchen homologen oder aber im wesentlichen homologen DNA Abschnitten verknüpft wird, **wodurch das einzuschleusende genetische Material von besagten homologen oder aber im wesentlichen homologen DNA Abschnitten flankiert wird;**
in die Myxobakterienzelle einschleust und dort via homologer Rekombination an einer aufgrund der zwischen eingeschleuster DNA und bakterieneigener DNA vorhandenen Homologien genau definierten Stelle in das Chromosom besagter Myxobakterien integriert; und
(b) positive Transformanten mit Hilfe an sich bekannter Selektionsverfahren selektiert und als Reinkultur kultiviert.

29. Genetisch modifizierte Myxobakterienzelle hergestellt nach einem Verfahren gemäss einem der Ansprüche 1 bis **13** sowie **28**.

30. Genetisch modifizierte Myxobakterienzelle aus der Sorangium/Polyangium Gruppe enthaltend eine via homologer Rekombination ins Myxobakterien-Genom integrierte exogene DNA homologen und/oder heterologen Ursprungs.

## Claims

1. Process for the genetic manipulation of myxobacteria from the *Sorangium/Polyangium* group,
characterised in that
(a) genetic material of homologous or heterologous origin or a combination of genetic material of homologous and heterologous origin, which is homologous with or else at least essentially homologous with a corresponding region on the myxobacterial chromosome; or else
(b) genetic material which naturally contains no sections which are homologous with or else at least essentially homologous with a corresponding region on the myxobacterial chromosome and is therefore artificially linked with the aid of rDNA techniques known per se to such homologous or else essentially homologous DNA sections, as a result of which the genetic material to be inserted is flanked by said homologous or else the essentially homologous DNA sections;
is inserted into the myxobacterial cell and there integrated, via homologous recombination, at a site, which is accurately defined by reason of the homologies present between inserted DNA and DNA which is intrinsic to the bacteria, into the chromosome of said myxobacteria.

2. Process according to claim 1, characterised in that said genetic material to be inserted is an expressable DNA which is linked in an operable manner to expression sequences able to function in the myxobacterial cell.

3. Process according to claim 1, characterised in that the insertion of the genetic material takes place via a donor organism capable of conjugation-like information exchange with the myxobacterium target organism from the donor to the myxobacterium recipient.

4. Process for the genetic manipulation of myxobacteria from the *Sorangium/Polyangium* group according to claim 1, which is characterised by the following process steps:
(a) preparation of the complete DNA of the myxobacterium target organism or of a related organism;
(b) fragmentation of the complete DNA isolated as in (a) ;
(c) cloning of the fragments as prepared in (b) into suitable plasmid vectors and transformation of said vectors into one of the host organisms normally used for cloning purposes;
(d) selection of those colonies which contain a plasmid with integrated myxobacterium DNA fragment and isolation of said plasmids;
(e) transformation of the plasmid DNA isolated as in (d) into a donor organism capable of conjugation-like information exchange with the myxobacterium target organism;
(f) conjugal transfer of the recombinant plasmid DNA to the myxobacterium target organism;
(g) cultivation of the transformed myxobacterium cells and selection of positive transformants.

5. Process according to either of claims 1 or 4, characterised in that the genetic material to be inserted is a plasmid which may contain one or more expressable DNA sections and which contains the homologous or else the essentially homologous DNA regions cloned in.

6. Process according to either of claims 1 or 4, characterised in that the degree of homology between said homologous DNA sections and the corresponding regions within the myxobacterial chromosome is between 80% and 100%.

7. Process according to either of claims 1 or 4, characterised in that said homologous DNA sections comprise at least 100 Bp.

8. Process according to either of claims 1 or 4, characterised in that said homologous DNA sections comprise between 0.3 and 4 Kb.

9. Process according to claim 4, characterised in that a prokaryote selected from the group consisting of *E. coli*, pseudomonads, actinomycetes, salmonellae and myxobacteria themselves is used as host organism for the cloning of the myxobacterium DNA fragments or a donor organism within the scope of the conjugal DNA transfer.

10. Process according to claim 4, characterised in that a restriction-negative or restriction-deficient bacterial strain is used as host organism for the cloning of the myxobacterium DNA fragments or as donor organism within the scope of the conjugal DNA transfer.

11. Process according to claim 11, characterised in that the cloning of the myxobacterium DNA fragments is carried out directly in a donor organism which is capable of conjugation-like information exchange with the myxobacterium target organism.

12. Process according to claim 4, characterised in that the myxobacterium cells are subjected to a brief heat treatment immediately before the conjugal DNA transfer.

13. Process according to claim 12, characterised in that the heat treatment is carried out at a temperature of 35°C to 60°C over a period of 1 to 120 minutes.

14. Recombinant DNA molecule which makes possible integration of genetic material at a defined site within the genome of myxobacteria from the *Sorangium/Polyangium* group, characterised in that said recombinant DNA molecule contains the DAN which is to be integrated, and in that said DNA has homologies with corresponding DNA regions within the myxobacterial genome, or else is flanked by such homologous DNA sequences, to an extent such that, on transformation of the myxobacterial cell containing the homologous DNA region, there is integration of said DNA, which is to be integrated, at a site, which is exactly defined by reason of the homology present between the inserted DNA and the DNA intrinsic to the bacteria, within the myxobacterial genome via homologous recombination.

15. Recombinant DNA molecule according to claim 14, characterised in that the degree of homology between said homologous DNA sections and the corresponding regions within the myxobacterial chromosome is between 80% and 100%.

16. Recombinant DNA molecule according to claim 14, characterised in that said homologous DNA sections comprise a region of at least 100 Bp.

17. Recombinant DNA molecule according to clam 14, characterised in that said DNA sequence to be integrated itself already has sufficiently great homology with corresponding DNA regions within the bacterial genome so that direct exchange of this DNA sequence for said homologous genomic DNA can take place by means of homologous recombination.

18. Recombinant DNA molecule according to claim 14, characterised in that said DNA to be inserted is double-stranded DNA.

19. Recombinant DNA molecule according to claim 14, characterised in that said DNA to be inserted is single-stranded DNA.

20. Recombinant DNA molecule according to claim 14, characterised in that said DNA to be inserted is an expressable DNA which is linked in an operable manner to expression sequences able to function in the myxobacterial cell.

21. Recombinant DNA molecule according to claim 14, characterised in that said flanking DNA sections are fused together to a unit, as component of a DNA molecule which is closed in the form of a ring.

22. Recombinant DNA molecule according to claim 14, characterised in that said homologous DNA sections originate from the myxobacterium genome itself.

23. Cloning vector containing a recombinant DNA molecule according to any of claims 14 to 22.

24. Plasmid DNA for the conjugal transfer from a donor organism to the myxobacterium recipient from the *Sorangium/Polyangium* group, characterised in that said plasmid DNA contains, besides the DNA to be inserted, homologous or else essentially homologous DNA sections, and transfer (tra) and mobilisation functions (mob) suitable for transfer into myxobacterium cells.

25. Plasmid DNA according to claim 24 containing a recombinant DNA molecule according to any of claims 14 to 22.

26. Process for the preparation of a recombinant DNA molecule according to claim 14, characterised in that the DNA which is to be integrated, which has homologies with corresponding DNA regions within the myxobacterial genome to an extent such that, on transformation of the myxobacterial genome containing the said homologous DNA region, there is integration of said DNA at a site, which is defined by reason of the homology present between the inserted DNA and the DNA intrinsic to the bacteria, within the bacterial genome via homologous recombination,
(a) is isolated from a source which contains homologous or else essentially homologous DNA sections; or
(b) when said DNA which is to be integrated naturally contains no sections which are homologous with or else at least essentially homologous with a corresponding region on the myxobacterial chromosome, this DNA is artificially linked with the aid of rDNA techniques known per se to corresponding homologous or else essentially homologous DNA sections.

27. Process according to claim 26, characterised in that said DNA to be inserted is located on a plasmid, and the homologous DNA sequences are cloned into the plasmid DNA at any desired site without, however, thereby destroying the functional integrity of the DNA to be inserted.

28. Process for the preparation of genetically modified myxobacteria from the *Sorangium/Polyangium* group according to claim 1, characterised in that
(a₁) genetic material of homologous or heterologous origin or a combination of genetic material of homologous and heterologous origin which is homologous with or else at least essentially homologous with a corresponding region on the myxobacterial chromosome; or else
(a₂) genetic material which naturally contains no sections which are homologous with or else at least essentially homologous with a corresponding region on the myxobacterial chromosome and is therefore artificially linked with the aid of rDNA techniques known per se to such homologous or else essentially homologous DNA sections, as a result of which the genetic material to be inserted is flanked by said homologous or else the essentially homologous DNA sections;
is inserted into the myxobacterial cell and there integrated, via homologous recombination, at a site, which is accurately defined by reason of the homologies present between inserted DNA and DNA which is intrinsic to the bacteria, into the chromosome of said myxobacteria; and
(b) positive transformants are selected with the aid of selection processes known per se and cultivated as pure culture.

29. Genetically modified myxobacterial cell prepared by a process according to any of claims 1 to 13 and 28.

30. Genetically modified myxobacterial cell from the Sorangium/Polyangium group containing an exogenous DNA of homologous and/or heterologous origin integrated into the myxobacterial genome via homologous recombination.

## Revendications

1. Procédé de manipulation génétique de myxobactéries du groupe *Sorangium/ Polyangium,* caractérisé en ce que :
(a) du matériel génétique d'origine homologue ou hétérologue ou une combinaison de matériel génétique d'origine homologue ou bien hétérologue, homologue ou au moins en grande partie homologue à une partie correspondante sur le chromosome myxobactérien ; ou
(b) du matériel génétique, qui ne contient pas naturellement de parties homologues ou au moins en grande partie homologues à un domaine correspondant du chromosome myxobactérien et est donc rattaché artificiellement à de telles portions d'ADN homologue ou en grande partie homologue, à l'aide de techniques d'ADNr connues par elles-mêmes, avec l'effet que le matériel génétique à introduire est flanqué par lesdites portions d'ADN homologue ou en grande partie homologue ;
est introduit dans la cellule myxobactérienne dans laquelle il est intégré via recombinaison homologue dans un site défini avec précision du fait des homologies présentes entre l'ADN introduit et l'ADN bactérien propre dans le chromosome desdites myxobactéries.

2. Procédé selon la revendication 1, caractérisé en ce que ledit matériel génétique introduit est un ADN exprimable, qui est rattaché de manière opérationnelle aux séquences d'expression fonctionnelle de la cellule myxobactérienne.

3. Procédé selon la revendication 1, caractérisé en ce que l'introduction du matériel génétique est effectuée par un organisme donneur compétent à effectuer un échange d'information de type conjugaison avec l'organisme-cible myxobactérien, du donneur vers la myxobactérie réceptrice.

4. Procédé de manipulation génétique de myxobactéries du groupe *Sorangium/ Polyangium* selon la revendication 1, caractérisé par les étapes de procédé suivantes :
(a) préparation de l'ADN total de l'organisme-cible myxobactérien ou d'un organisme apparenté ;
(b) fragmentation de l'ADN total isolé en (a) ;
(c) clonage des fragments préparés en (b) dans des plasmides-vecteurs adaptés et transformation desdits vecteurs dans un organisme-hôte utilisé habituellement pour le clonage
(d) sélection des colonies qui contiennent un plasmide ayant un fragment d'ADN myxobactérien intégré et isolation desdits plasmides ;
(e) transformation de l'ADN plasmidique isolé en (d) dans un organisme donneur compétent à effectuer un échange d'information de type conjugaison avec l'organisme-cible myxobactérien ;
(f) transfert par conjugaison de l'ADN plasmidique recombinant dans l'organisme cible myxobactérien ;
(g) culture des cellules myxobactériennes transformées et sélection des transformants positifs.

5. Procédé selon l'une des revendications 1 ou 4, caractérisé en ce que le matériel génétique introduit est un plasmide, qui contient le cas échéant une ou plusieurs portions d'ADN exprimables et comporte les parties d'ADN homologue ou en grande partie homologue obtenus par clonage.

6. Procédé selon l'une des revendications 1 ou 4, caractérisé en ce que le degré d'homologie entre lesdits domaines d'ADN homologues et les parties correspondantes sur le chromosome myxobactérien est compris entre 80 % et 100 %.

7. Procédé selon l'une des revendications 1 ou 4, caractérisé en ce que lesdits domaines d'ADN homologues contiennent au moins 100 pb.

8. Procédé selon l'une des revendications 1 ou 4, caractérisé en ce que lesdits domaines d'ADN contiennent entre 0,3 et 4 kb.

9. Procédé selon la revendication 4, caractérisé en ce que l'on utilise comme organisme-hôte pour le clonage des fragments d'ADN myxobactérien ou comme organisme donneur dans le cadre du transfert d'ADN par conjugaison, un procaryote choisi parmi le groupe constitué par *E. coli*, les Pseudomonas, les actinomycètes, les salmonelles ainsi que les myxobactéries elles-mêmes.

10. Procédé selon la revendication 4, caractérisé en ce que l'on utilise comme organisme-hôte pour le clonage des fragments d'ADN myxobactérien ou comme organisme donneur dans le cadre du transfert d'ADN par conjugaison, une souche bactérienne restriction-négative ou restriction-déficiente.

11. Procédé selon la revendication 10, caractérisé en ce que l'on effectue le clonage des fragments d'ADN myxobactérien directement dans un organisme donneur compétent pour un échange d'information de type conjugaison avec l'organisme-cible du myxobacterium.

12. Procédé selon la revendication 4, caractérisé en ce que l'on fait subir aux cellules myxobactériennes immédiatement avant le transfert d'ADN par conjugaison un bref traitement par la chaleur.

13. Procédé selon la revendication 12, caractérisé en ce que le traitement par la chaleur est effectué pendant un temps compris entre 1 et 120 minutes à une température comprise entre 35°C et 60°C.

14. Molécule d'ADN recombinant, permettant une intégration de matériel génétique en un site défini à l'intérieur du génome de myxobactéries du groupe *Sorangium/Polyangium,* caractérisée en ce que ladite molécule d'ADN recombinant contient l'ADN à intégrer, et en ce que ledit ADN présente un tel degré d'homologie avec les régions correspondantes d'ADN à l'intérieur du génome myxobactérien ou bien est flanquée d'une ou plusieurs de telles séquences d'ADN homologues, en ce qu'au moment de la transformation de la cellule contenant les régions d'ADN homologue, il se produit une intégration via une recombinaison homologue dudit ADN à intégrer en un site défini avec précision à l'intérieur du génome myxobactérien du fait des homologies existant entre l'ADN introduit et l'ADN bactérien propre.

15. Molécule d'ADN recombinant selon la revendication 14, caractérisée en ce que, le degré d'homologie entre lesdits domaines d'ADN homologue et les parties correspondantes à l'intérieur du chromosome myxobactérien est compris entre 80 et 100 %.

16. Molécule d'ADN recombinant selon la revendication 14, caractérisée en ce que lesdites portions d'ADN homologues contiennent une partie d'au moins 100 pb.

17. Molécule d'ADN recombinant selon la revendication 14, caractérisée en ce que ladite séquence d'ADN à intégrer présente elle-même une homologie avec les régions correspondantes d'ADN à l'intérieur du génome bactérien suffisamment importante, ce qui permet un échange direct de cette séquence d'ADN contre ledit ADN génomique homologue par recombinaison homologue.

18. Molécule d'ADN recombinant selon la revendication 14, caractérisée en ce que ledit ADN à introduire est un ADN double-brin.

19. Molécule d'ADN recombinant selon la revendication 14, caractérisée en ce que ledit ADN à introduire est un ADN simple-brin.

20. Molécule d'ADN recombinant selon la revendication 14, caractérisée en ce que, ledit ADN à introduire est un ADN exprimable, qui est rattaché d'une manière opérationnelle avec les séquences d'expression fonctionnelles dans la cellule myxobactérienne.

21. Molécule d'ADN recombinant selon la revendication 14, caractérisée en ce que, lesdites portions d'ADN flanquant, fusionnées entre elles en une unité, se présentent comme partie d'une molécule d'ADN fermée circulaire.

22. Molécule d'ADN recombinant selon la revendication 14, caractérisée en ce que, lesdites portions d'ADN homologue proviennent du génome myxobactérien lui-même.

23. Vecteur de clonage contenant une molécule d'ADN recombinant conformément à l'une revendications 14 à 22.

24. ADN plasmidique pour le transfert par conjugaison d'un organisme donneur aux myxobactéries receveuses du groupe *Sorangium/Polyangium*, caractérisé en ce que, ledit ADN plasmidique contient à côté de l'ADN à introduire des portions homologues ou en grande partie homologues ainsi que des fonctions de transfert (tra) et de mobilisation (mob) pour un transfert dans les cellules myxobactériennes.

25. ADN plasmidique selon la revendication 24, contenant une molécule d'ADN recombinant selon l'une des revendications 14 à 22.

26. Procédé de production d'une molécule d'ADN recombinant selon la revendication 14, caractérisé en ce que l'ADN à intégrer qui présente un degré d'homologie avec les régions correspondantes d'ADN à l'intérieur du génome myxobactérien, en ce qu'au moment de la transformation du génome myxobactérien contenant les dites régions d'ADN homologue, il se produit une intégration via une recombinaison homologue entre le dit ADN à intégrer et un site défini avec précision à l'intérieur du génome myxobactérien, du fait des homologies existant entre l'ADN introduit et l'ADN bactérien propre
(a) est isolée d'une source qui contient des domaines d'ADN homologues ou en grande partie homologues ; ou
(b) est artificiellement rattachée à l'aide d'une technique d'ADNr connue en elle-même avec les portions d'ADN homologues ou en grande partie homologues tant que ledit ADN à intégrer ne contient pas naturellement une portion homologue ou au moins en grande partie homologue à un domaine correspondant du chromosome myxobactérien.

27. Procédé selon la revendication 26, caractérisé en ce que, ledit ADN à introduire se trouve sur un plasmide et lesdites séquences d'ADN homologues sont clonées en un site choisi sur l'ADN plasmidique, sans détruire l'intégrité fonctionnelle de l'ADN à introduire.

28. Procédé de production de myxobactéries génétiquement modifiées du groupe *Sorangium/Polyangium* selon la revendication 1, caractérisé en ce que l'on introduit dans les cellules myxobactériennes
(a₁) du matériel génétique, d'origine homologue ou hétérologue ou bien une combinaison de matériel génétique d'origine homologue ainsi qu'hétérologue présentant des portions homologues ou en grande partie homologues à un domaine correspondant sur le chromosome myxobactérien ou bien
(a₂) du matériel génétique lequel ne contient pas naturellement des portions homologues ou en grande partie homologues à un domaine correspondant sur le chromosome myxobactérien et qui est donc rattaché artificiellement à l'aide de techniques d'ADNr connues en elles-mêmes avec de telles portions d'ADN homologues ou en grande partie homologues, de manière que le matériel génétique à introduire soit flanqué desdites portions d'ADN homologues ou en grande partie homologues ;
et qu'on intègre dans le chromosome desdites myxobactéries via une recombinaison homologue en un site défini avec précision du fait des homologies entre l'ADN introduit et l'ADN bactérien propre ; et
(b) que les transformées-positives sont sélectionnées par un procédé de sélection connu en lui-même et cultivées comme culture pure.

29. Cellules myxobactériennes génétiquement modifiées produites par un procédé selon l'une des revendications 1 à 13 ainsi que 28.

30. Cellules myxobactériennes du groupe Sorangium/Polyangium, génétiquement modifiées, contenant un ADN exogène d'origine homologue et/ou hétérologue intégré dans le génome myxobactérien via une recombinaison homologue.
